(19) Europäisches Patentamt
European Patent Office
Office européen des brevets

(11) Publication number: **0 194 750 A1**

# (12) EUROPEAN PATENT APPLICATION

(21) Application number: 86300883.5

(22) Date of filing: 10.02.86

(51) Int. Cl.[4]: C 07 D 211/90
C 07 D 405/12, C 07 D 401/12
C 07 D 417/12, C 07 D 413/04
A 61 K 31/44

(30) Priority: 11.02.85 GB 8503424

(43) Date of publication of application: 17.09.86 Bulletin 86/38

(84) Designated Contracting States: AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Hargreaves, Rodney Brian
42 Deva Close
Poynton Cheshire(GB)

(72) Inventor: McLoughlin, Bernard Joseph
7 Pexhill Drive
Macclesfield Cheshire(GB)

(72) Inventor: Mills, Stuart Dennett
17 Harrington Drive
Gawsworth Macclesfield SK11 9RD(GB)

(74) Representative: Slatcher, Reginald Peter et al,
Imperial Chemical Industries PLC Legal Department: Patents P.O. Box 6 Bessemer Road
Welwyn Garden City AL7 1HD(GB)

## (54) Dihydropyridine alkanol amines, process for their preparation and pharmaceutical compositions containing them.

(57) Dihydropyridines of the formula:-

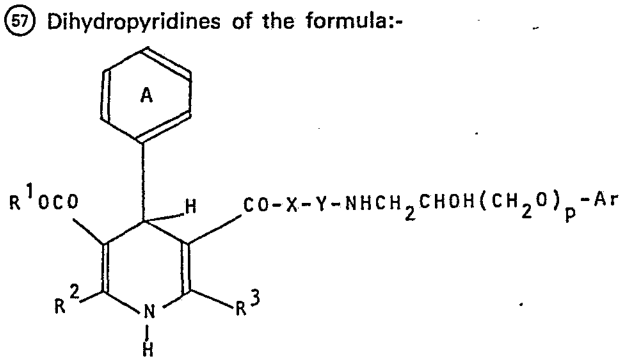

wherein $R^1$ is alkyl or alkoxyalkyl, wherein $R^2$ and $R^3$ each is alkyl, wherein benzene ring A bears one or more substituents selected from halogeno, cyano, nitro, trifluoromethyl and alkyl or bears the substituent =N–O–N= attached to the 2- and 3- positions, wherein Ar is phenyl, naphthyl, tetrahydronaphthyl, indanyl or indenyl which is unsubstituted or which bears one or more substituents, or Ar is a 5- or 6-membered heterocyclic ring containing 1, 2 or 3 heteroatoms selected from oxygen, nitrogen and sulphur, which ring is saturated or unsaturated, which ring is unsubstituted or bears one or more substituents, and which ring may also be fused to a benzene ring,

wherein p is 0 or 1,

wherein X is –O– or –NH–,

and wherein Y is straight-or branched-chain alkylene of 2 to 12 carbon atoms which may optionally be interrupted by one or two groups selected from oxygen, sulphur, imino, substituted imino, phenylene, substituted phenylene, pyridylene, cycloalkylene, 1,4-piperazinediyl, 1-4-piperidinediyl and amido groups, or an acid-addition salt thereof; processes for their manufacture and pharmaceutical compositions containing them. The compounds possess either beta-adrenergic blocking or calcium ion slow channel blocking properties, or both such properties, and may be used in the treatment of hypertension.

EP 0 194 750 A1

Croydon Printing Company Ltd

# HETEROCYCLIC DERIVATIVES

This invention relates to new heterocyclic derivatives and more particularly it relates to new dihydropyridine derivatives which possess antihypertensive properties.

Many 2,6-dialkyl-4-aryl-1,4-dihydropyridine-3,5-dicarboxylate derivatives are known which inhibit the movement of calcium ions in the cardiovascular system of warm-blooded animals, and which thereby produce an antihypertensive effect. The most-used of these is nifedipine, which is dimethyl 1,4-dihydro-2,6-dimethyl-4-o-nitrophenylpyridine-3,5-dicarboxylate.

Also known are many 1-aryloxy-3-amino-propan-2-ol derivatives which possess beta-adrenergic receptor blocking properties and which also produce an antihypertensive effect. Two of the most-used of these are propranolol and atenolol, which are respectively 1-(naphth-1-yloxy)- and 1-p-carbamoylmethylphenoxy-3-isopropylaminopropan-2-ol.

The only described attempt to combine these two types of chemical structure into one molecule is reported by Merck workers in the Journal of Medicinal Chemistry, 1981, Vol. 24, pages 628 to 631, in which a 3-amino-2-hydroxypropoxy substituent was introduced into the 4-aryl substituent of a 4-aryl-1,4-dihydropyridine derivative, without much success in producing a compound with antihypertensive activity of the type sought by the authors.

Since the priority date of the present application two documents have been published which describe closely related compounds to those of the present invention. These are European Specification No. 151066 in the name of the Yamanouchi Pharmaceutical Co.Ltd., and Japanese Specification No. 60/136558

(Derwent Abstract Reference No. 85-213756/35) in the name of Teikoku Hormone Manufacturing Ltd.

We have now found that compounds which do possess useful antihypertensive activity may be obtained by suitably combining a 3-aryloxy-2-hydroxypropylamino moiety with a 1,4-dihydropyridine moiety.

According to the present invention there is provided a dihydropyridine of the formula:

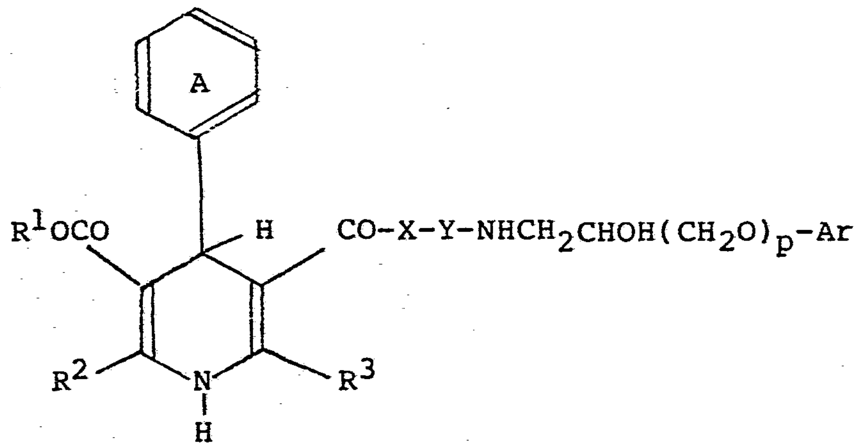

wherein $R^1$ is alkyl or alkoxyalkyl each of up to 6 carbon atoms:
wherein $R^2$ and $R^3$, which may be the same or different, each is alkyl of up to 6 carbon atoms;
wherein benzene ring A bears one or more substituents selected from halogeno, cyano, nitro, trifluoromethyl and alkyl of up to 6 carbon atoms or bears the substituent

$$=N-O-N=$$

attached to the 2- and 3-positions (that is, to form a benzo-2,1,3-oxadiazole nucleus);
wherein Ar is phenyl, naphthyl, tetrahydronaphthyl, indanyl or indenyl which is unsubstituted or which bears one or more substituents selected from halogeno, trifluoromethyl, hydroxy, amino, nitro, carbamoyl and cyano, and alkyl, alkenyl, alkoxy, alkenyloxy,

alkoxyalkoxy, alkylthio, alkanoyl, carbamoylalkyl and alkanoylamino each of up to 6 carbon atoms;
or Ar is a 5- or 6-membered heterocyclic ring containing 1, 2 or 3 heteroatoms selected from oxygen, nitrogen and sulphur, which ring is saturated or unsaturated, which ring is unsubstituted or bears one or more substituents selected from oxo, halogeno, trifluoromethyl, phenyl and morpholino, and alkyl and alkoxy each of up to 6 carbon atoms, and arylalkyl of up to 12 carbon atoms, and which ring may also be fused to a benzene ring, Ar being joined to the rest of the molecule either from the heterocyclic ring or from the fused benzene ring;
wherein p is 0 or 1;
wherein X is -O- or -NH-;
and wherein Y is straight-or branched-chain alkylene of 2 to 12 carbon atoms which may optionally be interrupted by one or two groups selected from oxygen (-O-), sulphur (-S-), imino and substituted imino (-$NR^4$ wherein $R^4$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;
or an acid-addition salt thereof.

It will be observed that the dihydropyridine derivative of the invention possesses at least two asymmetric carbon atoms, namely the carbon atom of the -CHOH- group in the alkanolamine chain, and the carbon atom at the 4-position of the dihydropyridine nucleus, and it can therefore exist in racemic and optically-active forms. It is to be understood that this invention encompasses the racemic form of the dihydropyridine derivative and any optically-active form which possesses antihypertensive activity, it being a

matter of common general knowledge how a racemic compound may be resolved into optically-active forms, and how the antihypertensive activity of these forms may be determined. It is further to be understood that beta-adrenergic blocking activity usually predominates in that optically-active form which has the "S" absolute configuration of the said -CHOH- group in the alkanolamine chain when p is 1, and the "R" absolute configuration when p is 0.

A suitable value for $R^1$, $R^2$, $R^3$, $R^4$ or a substituent in benzene ring A or Ar when it is alkyl is, for example, methyl, ethyl or isopropyl.

A suitable value for $R^1$ when it is alkoxyalkyl is, for example, methoxyethyl, ethoxyethyl or propoxyethyl.

A suitable halogeno substituent in the benzene ring A or in Ar is, for example fluoro, chloro or bromo.

A suitable value for the alkenyl, alkoxy, alkenyloxy, alkoxyalkoxy, alkylthio, carbamoylalkyl or alkanoylamino substituent in Ar is, for example, allyl, methoxy, ethoxy, isopropoxy, allyloxy, methoxyethoxy, methylthio, carbamoylmethyl or acetamido.

A suitable value for $R^4$ when it is alkanoyl, or for an alkanoyl substituent in Ar is, for example,formyl, acetyl or benzoyl.

A suitable value for $R^4$ when it is aralkyl, or for an aralkyl substituent in Ar is, for example, benzyl.

A suitable value for Ar when it is heterocyclic, either single ring or benzo-fused ring, is, for example, 4-morpholino-1,2,5-thiadiazol-3-yl, 1-methyl-4-indolyl, 2-oxo-1,2,3,4-tetrahydro-5-quinolyl, 4-indolyl, 4-carbazolyl, 4-benzo[b]thienyl, 5-benzo[1,4]dioxanyl, 3-cyano-2-pyridyl or 4-oxochroman-8-yl.

A suitable value for Y is, for example, straight-chain alkylene of the formula $(-CH_2)_n-$, wherein n is an integer from 2 to 12;

or $-(CH_2)_mC(CH_3)_2-$;

or $-(CH_2)_m-NH-(CH_2)_n-$

$-(CH_2)_m-N(CH_3)-(CH_2)_n-$

$-(CH_2)_m-O-(CH_2)_n-$

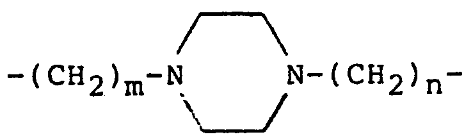

$-(CH_2)_m-CONH-(CH_2)_n-$

wherein m and n, which may be the same or different, each is 2,3,4 or 5;

or 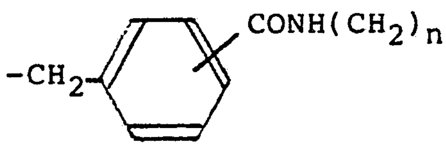

$-CH_2CONH(CH_2)_n-$

wherein n is 2, 3, 4 or 5;

or 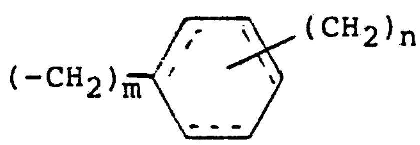 or 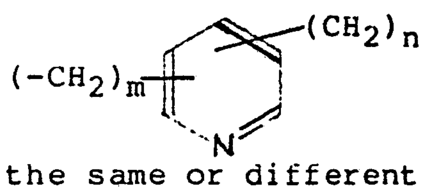

and wherein m and n, which may be the same or different, each is 1, 2, 3 or 4 and wherein the double bonds in the carbocyclic ring are optional (that is, cyclohexylene- or phenylene- bis-alkylene).

A suitable acid-addition salt of a dihydropyridine derivative of the invention is, for example, a salt derived from an inorganic acid, for example a hydrochloride, hydrobromide, phosphate or sulphate, or a salt derived from an organic acid, for example an oxalate, lactate, succinate, tartrate, acetate, salicylate, citrate, benzoate, beta-naphthoate or adipate.

One preferred dihydropyridine of the invention has the formula stated above wherein $R^1$ is alkyl or alkoxyalkyl each of up to 4 carbon atoms, wherein $R^2$ and $R^3$ are both methyl, wherein benzene ring A bears one or two substituents selected from fluoro and chloro, or bears one cyano, nitro, trifluoromethyl or methyl substituent, or the 2,3 =N-O-N= substituent, wherein Ar is phenyl which is unsubstituted or which bears one or two chloro or methyl substituents, or one fluoro, chloro, nitro, carbamoyl, cyano, methyl, methoxy, ethoxy, methoxyethoxy, acetyl, carbamoylmethyl or acetamido substituent, or Ar is unsubstituted naphthyl or tetrahydronaphthyl, or Ar is 1,4-benzodioxan-5-yl, 4-morpholino-1,2,5-thiadiazol-3-yl, 4-indolyl or 4-oxochroman-8-yl;
wherein p is 0 or 1, wherein X is -NH- and wherein Y is straight-chain alkylene of 2 to 12 carbon atoms, or branched-chain alkylene of 2 to 6 carbon atoms, or straight-chain alkylene of 2 to 12 carbon atoms which is interrupted by one oxygen, imino, alkylimino, aralkylamino, phenylene, cyclohexylene or amido group, or which is interrupted by two oxygen groups, or is an acid-addition salt thereof.

A second preferred dihydropyridine of the invention has the formula stated above wherein $R^1$, $R^2$, $R^3$, ring A and Ar have the meanings stated in the last paragraph above, wherein p is 1, wherein X is -O- and wherein Y is straight-chain alkylene of 2 to 12 carbon atoms which is interrupted by one imino, alkylimino, aralkylimino, phenylene, cyclohexylene or amido group, or is an acid-addition salt thereof.

A particularly preferred dihydropyridine of the invention is as defined in either of the last two paragraphs above wherein $R^1$ is methyl or ethyl, ring A bears a 2- or 3- nitro or a 2-fluoro, 2-chloro, 2-trifluoromethyl or 2-methyl substituent, Ar is phenyl which is unsubstituted or bears a 2-chloro, 2-cyano or 2-methoxy substituent, or Ar is unsubstituted naphthyl or 1,4-benzodioxan-5-yl, and p is 1.

Specific dihydropyridine derivatives of the invention are hereinafter described in the Examples. Of these, preferred compounds are:-

methyl 1,4-dihydro-2,6-dimethyl-4-m-nitrophenyl-5-[3-{N-(2-[2-hydroxy-3-phenoxypropylamino]ethyl)carbamoyl}-propoxycarbonyl]pyridine-3-carboxylate;

methyl 5-N-[5-(3-o-cyanophenoxy-2-hydroxypropylamino)-pentyl]carbamoyl-1,4-dihydro-2,6-dimethyl-4-m-nitrophenylpyridine-3-carboxylate;

methyl 4-o-chlorophenyl-1,4-dihydro-5-N-[5-(2-hydroxy-3-o-methoxyphenoxypropylamino)pentyl]carbamoyl-2,6-dimethylpyridine-3-carboxylate;

methyl 1,4-dihydro-5-N-[5-(2-hydroxy-2-o-methoxyphenyl-ethylamino)pentyl]carbamoyl-2,6-dimethyl-4-m-nitrophenylpyridine-3-carboxylate;

methyl 4-o-chlorophenyl-1,4-dihydro-5-N-[3-(2-hydroxy-3-phenoxypropylaminomethyl)benzyl]carbamoyl-2,6-dimethylpyridine-3-carboxylate;

methyl 4-(2-chloro-6-fluorophenyl)-5-N-[3-(3-o-cyano-phenoxy-2-hydroxypropylaminomethyl)benzyl]carbamoyl-1,4-dihydro-2,6-dimethylpyridine-3-carboxylate;

2-{3-[2-(2-hydroxy-3-phenoxypropylamino)ethyl]phenyl}-ethyl 4-o-chlorophenyl-1,4-dihydro-5-methoxycarbonyl-2,6-dimethylpyridine-3-carboxylate; and

4-[N-5-(3-o-cyanophenoxy-2-hydroxy-propylamino)pentyl-carbamoyl]-butyl 1,4-dihydro-5-methoxycarbonyl-2,6-dimethyl-4-m-nitrophenylpyridine-3-carboxylate.

The dihydropyridine derivatives of the invention may be manufactured by any chemical process known to be useful for the manufacture of chemically-analogous compounds.

One preferred process for the manufacture of a dihydropyridine derivative of the invention comprises the reaction of an amine of the formula:-

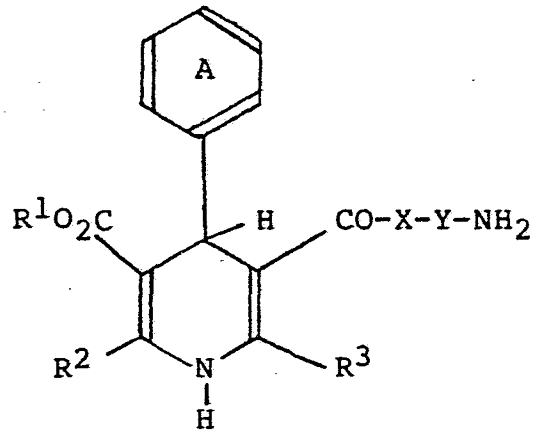

wherein A, $R^1$, $R^2$, $R^3$, X and Y have the meanings stated above, with an epoxide of the formula:-

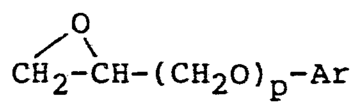

wherein Ar and p have the meanings stated above, or when p is 0, with a haloketone of the formula

$$HalCH_2CO\text{-}Ar$$

wherein Ar has the meaning stated above and where Hal stands for a halogeno group, for example bromo, followed by reduction, for example with sodium borohydride, of the aminoketone thus obtained.

The reaction may be carried out in an alcoholic diluent or solvent, for example in isopropanol, at a temperature of up to the boiling point of said diluent or solvent.

A second preferred process for the manufacture of a dihydropyridine derivative of the invention wherein the group -Y- is alkylene interrupted as stated above comprises joining the two parts of the molecule at the point of interruption of Y. Thus, for example, when Y is alkylene interrupted by an amido group -CONH-, the

process comprises the reaction of an acid of the formula:-

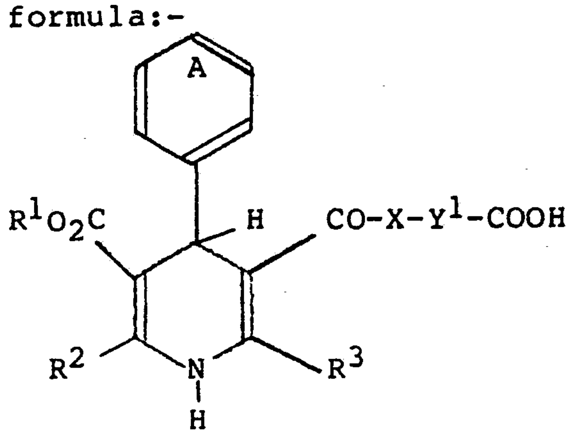

wherein A, $R^1$, $R^2$, $R^3$ and X have the meanings stated above, or an activated derivative thereof, with an amine of the formula:-

$$H_2N\text{-}Y^2\text{-}NHCH_2CHOH(CH_2O)_pAr$$

wherein Ar and p have the meanings stated above, and wherein $Y^1$ and $Y^2$ are such that $-Y^1\text{-}CONH\text{-}Y^2-$ has the same meaning as stated above for Y.

A thira process for the manufacture of a dihydropyridine of the invention comprises the reaction of an acid of the formula:-

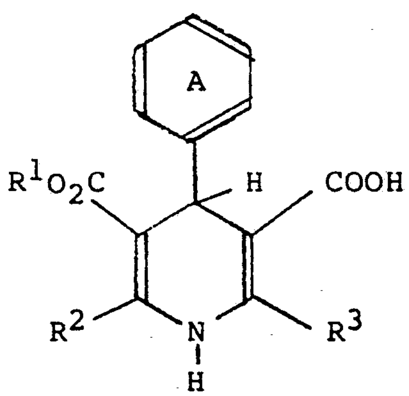

wherein A, $R^1$, $R^2$ and $R^3$ have the meanings stated above, or an activated derivative thereof, with an alcohol or

amine of the formula:-

$$H-X-Y-NHCH_2CHOH(CH_2O)_p-Ar$$

wherein Ar, p, X and Y have the meanings stated above.

A fourth process for the manufacture of a dihydropyridine of the invention comprises the reaction of a compound of the formula:-

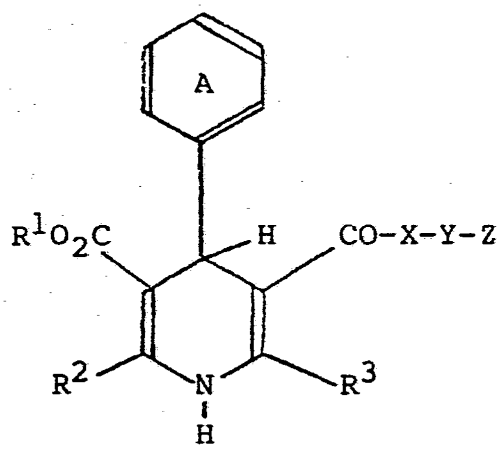

wherein A, $R^1$, $R^2$, $R^3$, X and Y have the meanings stated above and wherein Z stands for a displaceable group, with an amine of the formula:-

$$H_2NCH_2CHOH(CH_2O)_p-Ar$$

wherein Ar and p have the meanings stated above.

A suitable value for Z is, for example, a halogeno group, for example a bromo or chloro group, or a sulphonyloxy group, for example a methanesulphonyloxy or *p*-toluenesulphonyloxy group.

A fifth process for the manufacture of a dihydropyridine of the invention comprises the reaction of an aldehyde of the formula

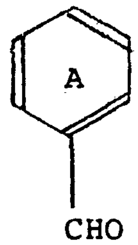

wherein A has the meaning stated above, an aminocrotonate of the formula

$$R^2-\overset{NH_2}{\overset{|}{C}}=CH-CO_2R^1$$

wherein $R^1$ and $R^2$ have the meanings stated above and a ketoacid derivative of the formula

$$R^3COCH_2CO-X-Y-NHCH_2CHOH(CH_2O)_p-Ar$$

wherein Ar, p, $R^3$, X and Y have the meanings stated above.

This process may be carried out in a diluent or solvent at an elevated temperature, conditions conventionally used for the Hantszch synthesis of dihydropyridines.

As stated above, the dihydropyridine derivatives of the invention possess antihypertensive activity. This may be demonstrated by the ability of the compound to reduce the blood pressure of a spontaneously hypertensive rat, or of a rat made hypertensive by treatment with deoxycorticosterone acetate, or of a dog made hypertensive by the Goldblatt technique of unilateral nephrectomy and clipping of the contralateral kidney. These are all standard tests used to demonstrate antihypertensive effects of medicaments.

Some of the dihydropyridine derivatives of the invention possess beta-adrenergic blocking properties, some of them possess calcium ion slow-channel blocking

properties and some of them possess both such activities. A preferred dihydropyridine derivative of the invention possesses both such activities. Beta-adrenergic blocking activity may be demonstrated *in vivo* by the ability of the compound to inhibit isoprenaline-induced tachycardia in a rat or cat, or *in vitro* by shifting to the right the dose- response curve of a guinea pig atrium to isoprenaline. Calcium ion slow channel blocking activity may be demonstrated *in vitro* by the ability of the compound to reduce spontaneous contraction in a rat portal vein preparation. These also are all standard tests used to demonstrate the stated activities.

Because of the beta-adrenergic blocking and/or calcium slow channel blocking properties a dihydropyridine of the invention may also be useful in the treatment of heart diseases such as angina pectoris and cardiac arrhythmias.

At doses of a dihydropyridine derivative which produce effective antihypertensive activity in a rat or dog no symptom of toxicity is apparent.

The dihydropyridine derivative of the invention may be administered to warm-blooded animals, including man, in the form of a pharmaceutical composition comprising as active ingredient at least one dihydropyridine derivative of the invention, or an acid-addition salt thereof, in association with a pharmaceutically-acceptable diluent or carrier therefor.

A suitable composition is, for example, a tablet, capsule, aqueous or oily solution or suspension, dispersible powder, spray or aerosol formulation.

The pharmaceutical composition may contain, in addition to the dihydropyridine derivative of the invention, one or more drugs selected from sedatives,

for example phenobarbitone, meprobamate, chloropromazine and the benzodiazepine sedative drugs, for example chlordiazepoxide and diazepam; vasodilators, for example glyceryl trinitrate, pentaerythritol tetranitrate, isosorbide dinitrate and hydralazine; diuretics, for example chlorthalidone, bendrofluazide, hydrochlorothiazide and chlorothiazide; other antihypertensive agents, for example reserpine, bethanidine and guanethidine; cardiac membrane stabilising agents, for example quinidine; agents used in the treatment of Parkinson's disease and other tremors, for example benzhexol; cardiotonic agents, for example digitalis preparations; and alpha-adrenergic blocking agents, for example phentolamine.

When used for the treatment of heart diseases, for example angina pectoris and cardiac arrhythmias, or for the treatment of hypertension in man, it is expected that the dihydropyridine derivative would be given to man at a total oral dose of between 20 mg. and 600 mg. daily, or at an intravenous dose of between 1 mg. and 20 mg.

Preferred oral dosage forms are tablets or capsules containing between 10 and 100 mg., and preferably 10 mg. or 50 mg., of active ingredient. Preferred intravenous dosage forms are sterile solutions of the dihydropyridine derivative or of a non- toxic acid-addition salt thereof, containing between 0.05% and 1% w/v of active ingredient, and more particularly containing 0.1% w/v of active ingredient.

The invention is illustrated but not limited by the following Examples:-

Example 1

A stirred mixture of 4-[1,4-dihydro-5-methoxycarbonyl-2,6-dimethyl-4-*m*-nitrophenylpyrid-3-yl-carbonyloxy]butyric acid (1.46 g.), 1-(2-aminoethylamino)-3-phenoxypropan-2-ol (1.05 g.), 2-ethoxy-1-ethoxycarbonyl-1,2-dihydroquinoline (0.86 g.) and dimethyl sulphoxide (25 ml.) was heated at 60°C. for 24 hours, diluted with water (500 ml.), basified with saturated aqueous sodium bicarbonate solution (20 ml.) and extracted three times with ethyl acetate (100 ml. each time). The combined extracts were dried over magnesium sulphate and evaporated to dryness, and the residue was purified by flash chromatography on a silica gel column (Merck 9385, 200 g.) using a 25:25:1 v/v/v mixture of ethyl acetate, methanol and acetic acid as eluent. There was thus obtained as an oil methyl 1,4-dihydro-2,6-dimethyl-4-*m*-nitrophenyl-5-[3-{*N*-(2-[2-hydroxy-3-phenoxypropylamino]ethyl)carbamoyl}-propoxycarbonyl]pyridine-3-carboxylate hemihydrate, the structure of which was confirmed by elemental analysis and proton magnetic resonance spectroscopy.

Elemental analysis

Found: C,60.1%; H, 6.4%; N, 8.9%.$C_{30}H_{38}N_4O_9$. ½$H_2O$ requires C,60.1%; H, 6.3%; N,9.0%.

Proton magnetic resonance spectrum (in $CDCl_3$)

| Shift (δ) | Type | No of protons | Assignment |
|---|---|---|---|
| 1.8-2.3 | broad | 4 | C-$(CH_2)$-C |
| 2.4 | singlet | 6 | $CH_3$ |
| 2.8-3.0 | broad | 4 | C-$(CH_2)$-N |
| 3.2-3.5 | quartet | 2 | $CH_2$-NHCO |
| 3.65 | singlet | 3 | $COOCH_3$ |
| 3.8-4.3 | broad | 5 | C-$CH_2$-O |
| | | | CHOH |
| 5.1 | singlet | 1 | pyridine 4H |
| 6.0 | singlet | 1 | pyridine NH |
| 6.2 | triplet | 1 | amide NH |
| 6.8-8.2 | complex | 9 | aromatic |

The butyric acid used as starting material was obtained as follows:-

A vigorously stirred mixture of 1,4-dihydro-5-methoxycarbonyl-2,6-dimethyl-4-m-nitrophenylpyridine-3-carboxylic acid (8.3 g.), ethyl 4-bromobutyrate (4.9 g.), anhydrous potassium carbonate (3.5 g.) and acetone (200 ml.) was heated under reflux for 18 hours, filtered and the filtrate was evaporated to dryness under reduced pressure. Aqueous 3N-sodium hydroxide solution (8.3 ml.) was added to a solution of the residue in ethanol (100 ml.) and the mixture was stirred at laboratory temperature for 18 hours and then evaporated to dryness under reduced pressure. The residue was partitioned between water and ethyl acetate and the aqueous solution was separated, acidified with aqueous N-hydrochloric acid and extracted three times with ethyl acetate (80 ml. each time). The combined extracts were dried over magnesium sulphate and evaporated to dryness and the residue was crystallised from methanol. There was thus obtained 4-[1,4-dihydro-5-methoxycarbonyl-2,6-dimethyl-4-m-nitrophenylpyrid-3-ylcarbonyloxy]butyric acid, m.p. 162-165°C.

Example 2

A mixture of 4-[1,4-dihydro-5-methoxycarbonyl-2,6-dimethyl-4-m-nitrophenylpyrid-3-ylcarbonyloxy]butyric acid (2.09 g.), 1-(2-aminoethylamino)-3-phenoxypropan-2-ol (1.05 g.), 1-hydroxybenzotriazole (0.84 g.), dicyclohexylcarbodiimide (1.13 g.) and methylene chloride (500 ml.) was stirred at laboratory temperature for 18 hours, cooled to 0°C. and filtered. The filtrate was washed with saturated aqueous sodium bicarbonate solution (100 ml.) and then with water (100 ml.), dried over magnesium sulphate and evaporated to dryness. The residue was purified by

flash chromatography on a silica gel column (Merck 9385, 200 g.) using a 90:10:3 v/v/v mixture of ethyl acetate, methanol and concentrated aqueous ammonia solution as eluant. There was thus obtained as an oil methyl 1,4-dihydro-2,6-dimethyl-4-m-nitrophenyl-5-[3- N-(2-[2-hydroxy-3-phenoxypropylamino]ethyl)carbamoyl - propoxycarbonyl]pyridine-3-carboxylate, identical to the product described in Example 1.

The process described above was repeated using the appropriate dihydropyridine-3-ylcarbonyloxy-alkanoic acid and the appropriate 1-(omega-aminoalkylamino)-3-aryloxypropan-2-ol as starting materials and there were thus obtained as oils the compounds described in the following table, the structures of all of which were confirmed by elemental analysis and proton magnetic resonance spectroscopy:-

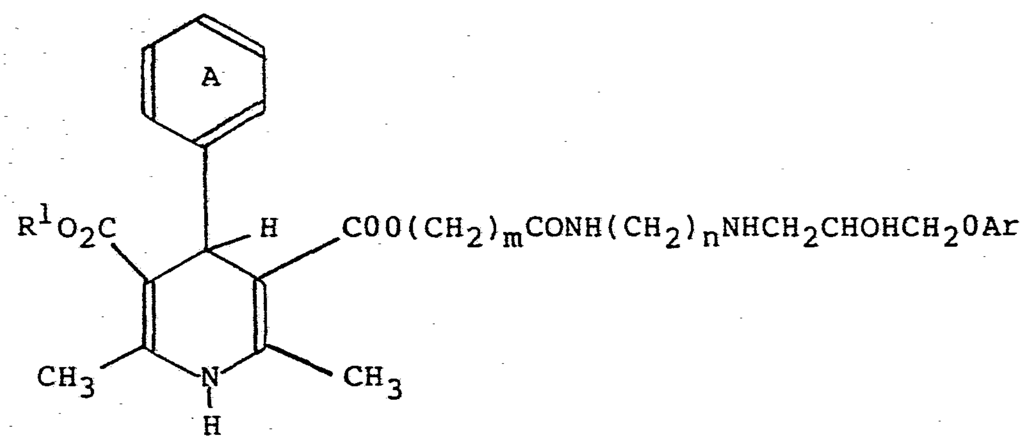

| $R^1$ | Ring A Substituent | m | n | Ar |
|---|---|---|---|---|
| Me | $3-NO_2$ | 1 | 2 | 2-cyanophenyl |
| Me | $3-NO_2$ | 1 | 3 | phenyl |
| Me | $3-NO_2$ | 1 | 3 | 2-cyanophenyl |
| Me | $3-NO_2$ | 3 | 2 | 2-chlorophenyl |
| Me | $3-NO_2$ | 3 | 2 | 2-methoxyphenyl |
| Me | $3-NO_2$ | 3 | 3 | phenyl |
| Me | $3-NO_2$ | 3 | 3 | 2-cyanophenyl |
| Me | $3-NO_2$ | 3 | 4 | phenyl |
| Me | $3-NO_2$ | 3 | 4 | 2-cyanophenyl |
| Me | $3-NO_2$ | 3 | 4 | 2-methoxyphenyl |
| Me | $3-NO_2$ | 3 | 5 | phenyl |
| Me | $3-NO_2$ | 3 | 5 | 2-cyanophenyl |
| Me | $3-NO_2$ | 4 | 2 | phenyl |
| Me | $3-NO_2$ | 4 | 2 | 2-cyanophenyl |
| Me | $3-NO_2$ | 4 | 2 | 2-fluorophenyl |
| Me | $3-NO_2$ | 4 | 2 | 2-chlorophenyl |
| Me | $3-NO_2$ | 4 | 2 | 2-methoxyphenyl |
| Me | $3-NO_2$ | 4 | 4 | phenyl |
| Me | $3-NO_2$ | 4 | 5 | phenyl |
| Me | $3-NO_2$ | 4 | 5 | 2-cyanophenyl |
| Et | $2-CF_3$ | 3 | 5 | 2-cyanophenyl |
| Me | $2-CH_3$ | 3 | 2 | phenyl |
| Me | $2-CH_3$ | 3 | 2 | 2-cyanophenyl |

The alkanoic acid starting materials were prepared by a similar process to that described in the second part of Example 1 from the appropriate dihydropyridine-3-carboxylic acid and the appropriate halogenoalkanoate ester. Those intermediates which were characterised by melting point have the formula:

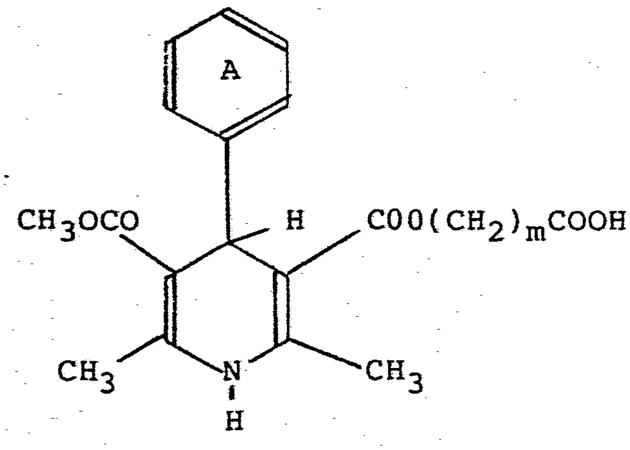

| Substituent in Ring A | m | m.p. (°C.) |
|---|---|---|
| 3-$NO_2$ | 1 | 181-183 |
| 3-$NO_2$ | 4 | 147-150 |
| 2-$CH_3$ | 3 | 127-130 |

Example 3

A solution of methyl 5-(N-5-aminopentyl-carbamoyl)-1,4-dihydro-2,6-dimethyl-4-m-nitrophenyl-pyridine-3-carboxylate (2.08 g.) and 1-o-cyanophenoxy-2,3-epoxypropane (0.87 g.) in isopropanol (20 ml.) was heated under reflux for 4 hours and then evaporated to dryness under reduced pressure. The residue was purified by flash chromatography on a silica gel column (Merck 9385) using an 80:20:3 v/v/v mixture of ethyl acetate, ethanol and triethylamine as eluent. There

was thus obtained as an oil methyl 5-*N*-[5-(3-*o*-cyanophenoxy-2-hydroxypropylamino)pentyl]carbamoyl-1,4-dihydro-2,6-dimethyl-4-*m*-nitrophenylpyridine-3-carboxylate, the structure of which was confirmed by elemental analysis and proton magnetic resonance spectroscopy.

Elemental analysis

Found: C, 60.3%; H, 6.3%; N,11.7%. $C_{31}H_{37}N_5O_7$ requires C, 60.2%; H,6.5%; N,11.3%.

Proton magnetic resonance spectrum (in $CDCl_3$)

| Shift (δ) | Type | No.of protons | Assignment |
|---|---|---|---|
| 1.0-1.7 | broad | 6 | $C-(CH_2)-C$ |
| 2.2 | singlet | 3 | $CH_3$ |
| 2.3 | singlet | 3 | $CH_3$ |
| 2.6-3.3 | broad | 6 | $C-(CH_2)-N$ |
| 3.5-3.7 | broad | 3 | $COOCH_3$ |
| 4.0-4.3 | broad | 3 | $-OCH_2CH(OH)-$ |
| 4.9 | singlet | 1 | pyridine 4H |
| 5.8-6.0 | triplet | 1 | amide NH |
| 6.7-8.2 | complex | 9 | 8 aromatic, pyridine NH |

The carboxylate used as starting material was obtained as follows:-

Dicyclohexylcarbodiimide (22.7 g.) was added to a stirred mixture of 1,4-dihydro-5-methoxycarbonyl-2,6-dimethyl-4-*m*-nitrophenylpyridine-3-carboxylic acid (33.2 g.) and 1-hydroxybenzotriazole (14.85 g.) in dimethylformamide (300 ml.) and the mixture was stirred at laboratory temperature for 4 hours, filtered and the filtrate was evaporated to dryness. The residue was

stirred with ethyl acetate, the mixture was filtered and the filtrate was evaporated to dryness, and this procedure was repeated until no further solid was precipitated. The residue after the final evaporation crystallised and there was thus obtained benzotriazol-1-yl 1,4-dihydro-5-methoxycarbonyl-2,6-dimethyl-4-m-nitrophenylpyridine-3-carboxylate, m.p. 173-176°C.

A solution of the above compound (15.9 g.) in methylene chloride (50 ml.) was slowly added to a stirred, ice-cooled solution of pentamethylene-1,5-diamine (50 ml.), and the mixture was stirred and allowed to warm up to laboratory temperature during 2 hours and was then evaporated to dryness. The residue was purified by flash chromatography on a silica gel column (Merck 9385) using a 12:7:4:2 v/v/v/v mixture of toluene, ethanol, ethyl acetate and aqueous ammonia (specific gravity 0.88) as eluent. There was thus obtained, as an oil, methyl 5-(N-5-aminopentylcarbamoyl)-1,4-dihydro-2,6-dimethyl-4-m-nitrophenylpyridine-3-carboxylate.

Example 4

The process described in Example 3 was repeated using the appropriate 5-aminoalkylcarbamoyl-1,4-dihydropyridine and the appropriate aryloxyepoxypropane or aryloxirane as starting materials and there were thus obtained as oils the compounds described in the following table, the structures of all of which were confirmed by proton magnetic resonance spectroscopy and either by elemental analysis or by mass spectroscopy:-

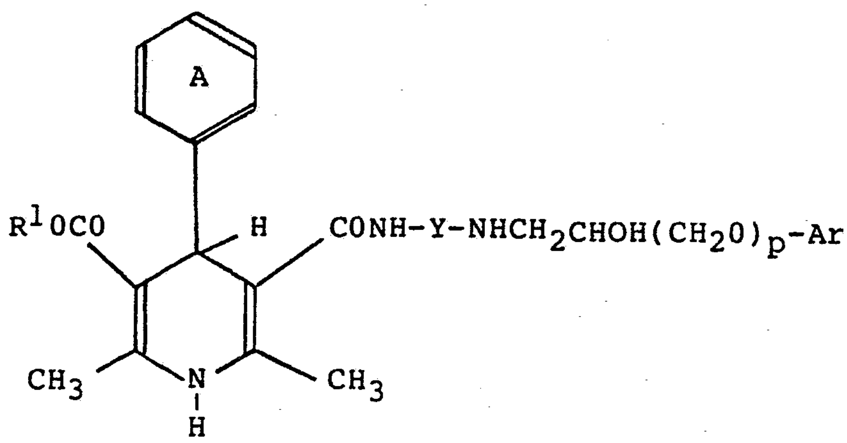

| $R^1$ | Ring A Substituent | Y | p | Ar |
|---|---|---|---|---|
| $CH_3$ | 3-$NO_2$ | $(CH_2)_2$ | 1 | 2-chlorophenyl |
| $CH_3$ | 3-$NO_2$ | $(CH_2)_2$ | 1 | 2-nitrophenyl |
| $CH_3$ | 3-$NO_2$ | $(CH_2)_2$ | 1 | 2-cyanophenyl |
| $CH_3$ | 3-$NO_2$ | $(CH_2)_2$ | 1 | 2-tolyl |
| $CH_3$ | 3-$NO_2$ | $(CH_2)_2$ | 1 | 2-methoxyphenyl |
| $CH_3$ | 3-$NO_2$ | $(CH_2)_2$ | 1 | 4-carbamoylmethyl-phenyl |
| $CH_3$ | 3-$NO_2$ | $(CH_2)_2$ | 1 | 1-naphthyl |
| $CH_3$ | 3-$NO_2$ | $(CH_2)_3$ | 1 | phenyl |
| $CH_3$ | 3-$NO_2$ | $(CH_2)_3$ | 1 | 2-cyanophenyl |
| $CH_3$ | 3-$NO_2$ | $(CH_2)_3$ | 1 | 1-naphthyl |
| $CH_3$ | 3-$NO_2$ | $(CH_2)_4$ | 1 | phenyl |
| $CH_3$ | 3-$NO_2$ | $(CH_2)_4$ | 1 | 2-cyanophenyl |
| $CH_3$ | 3-$NO_2$ | $(CH_2)_4$ | 1 | 2-carbamoylphenyl |
| $CH_3$ | 3-$NO_2$ | $(CH_2)_4$ | 1 | 2-tolyl |
| $CH_3$ | 3-$NO_2$ | $(CH_2)_4$ | 1 | 2-methoxyphenyl |
| $CH_3$ | 3-$NO_2$ | $(CH_2)_4$ | 1 | 2-acetylphenyl |
| $CH_3$ | 3-$NO_2$ | $(CH_2)_4$ | 1 | 4-(2-methoxyethoxy)-phenyl |

| $R^1$ | Ring A Substituent | Y | p | Ar |
|---|---|---|---|---|
| $CH_3$ | 3-$NO_2$ | $(CH_2)_5$ | 1 | 4-carbamoylmethyl-phenyl |
| $CH_3$ | 3-$NO_2$ | $(CH_2)_5$ | 1 | 2-acetamidophenyl |
| $CH_3$ | 3-$NO_2$ | $(CH_2)_5$ | 1 | 1,4-benzodioxan-5-yl |
| $CH_3$ | 3-$NO_2$ | $(CH_2)_5$ | 1 | 4-oxochroman-8-yl |
| $CH_3$ | 3-$NO_2$ | $(CH_2)_5$ | 1 | phenyl |
| $CH_3$ | 3-$NO_2$ | $(CH_2)_5$ | 1 | 2-cyanophenyl |
| $C_2H_5$ | 3-$NO_2$ | $(CH_2)_5$ | 1 | 2-cyanophenyl |
| $CH(CH_3)_2$ | 3-$NO_2$ | $(CH_2)_5$ | 1 | 2-cyanophenyl |
| $CH_3$ | 3-$NO_2$ | $(CH_2)_5$ | 1 | 2-tolyl |
| $CH_3$ | 3-$NO_2$ | $(CH_2)_5$ | 1 | 2-methoxyphenyl |
| $CH_3$ | 3-$NO_2$ | $(CH_2)_5$ | 1 | 1-naphthyl |
| $CH_3$ | 3-$NO_2$ | $(CH_2)_5$ | 1 | 4-indolyl |
| $CH_3$ | 3-$NO_2$ | $(CH_2)_5$ | 1 | 1,4-benzodioxan-5-yl |
| $C_2H_5O(CH_2)_2$ | 3-$NO_2$ | $(CH_2)_5$ | 1 | 1,4-benzodioxan-5-yl |
| $CH(CH_3)_2$ | 3-$NO_2$ | $(CH_2)_5$ | 1 | 1,4-benzodioxan-5-yl |
| $CH_3$ | 3-$NO_2$ | $(CH_2)_5$ | 1 | 4-morpholino-1,2,5-thiadiazol-3-yl |
| $CH_3$ | 3-$NO_2$ | $(CH_2)_6$ | 1 | phenyl |
| $CH_3$ | 3-$NO_2$ | $(CH_2)_6$ | 1 | 2-cyanophenyl |
| $CH_3$ | 3-$NO_2$ | $(CH_2)_6$ | 1 | 2-methoxyphenyl |
| $CH_3$ | 3-$NO_2$ | $(CH_2)_6$ | 1 | 1-naphthyl |
| $CH_3$ | 3-$NO_2$ | $(CH_2)_6$ | 1 | 1,4-benzodioxan-5-yl |
| $CH_3$ | 3-$NO_2$ | $(CH_2)_7$ | 1 | phenyl |
| $CH_3$ | 3-$NO_2$ | $(CH_2)_7$ | 1 | 2-cyanophenyl |
| $CH_3$ | 3-$NO_2$ | $(CH_2)_7$ | 1 | 1-naphthyl |
| $CH_3$ | 3-$NO_2$ | $(CH_2)_8$ | 1 | 2-cyanophenyl |
| $CH_3$ | 3-$NO_2$ | $(CH_2)_{12}$ | 1 | 2-cyanophenyl |
| $CH_3$ | 3-$NO_2$ | $CH_2C(CH_3)_2$ | 1 | phenyl |
| $CH_3$ | 3-$NO_2$ | $CH_2C(CH_3)_2$ | 1 | 2-cyanophenyl |
| $CH_3$ | 3-$NO_2$ | $CH_2C(CH_3)_2$ | 1 | 1-naphthyl |
| $CH_3$ | 3-$NO_2$ | $CH_2C(CH_3)_2$ | 1 | 4-indolyl |

| $R^1$ | Ring A Substituent | Y | p | Ar |
|---|---|---|---|---|
| $CH_3$ | 3-$NO_2$ | $(CH_2)_3O(CH_2)_3$ | 1 | 2-cyanophenyl |
| $CH_3$ | 3-$NO_2$ | $(CH_2)_3O(CH_2)_3$ | 1 | 2-methoxyphenyl |
| $CH_3$ | 3-$NO_2$ | $(CH_2)_3O(CH_2)_3$ | 1 | 1-naphthyl |
| $CH_3$ | 3-$NO_2$ | $(CH_2)_5O(CH_2)_5$ | 1 | 2-cyanophenyl |
| $CH_3$ | 3-$NO_2$ | $(CH_2)_5O(CH_2)_5$ | 1 | 2-methoxyphenyl |
| $CH_3$ | 3-$NO_2$ | $(CH_2)_2O(CH_2)_2O(CH_2)_2$ | 1 | 2-cyanophenyl |
| $CH_3$ | 3-$NO_2$ | $(CH_2)_2O(CH_2)_2O(CH_2)_2$ | 1 | 2-methoxyphenyl |
| $CH_3$ | 3-$NO_2$ | $(CH_2)_3O(CH_2)_4O(CH_2)_3$ | 1 | 2-cyanophenyl |
| $CH_3$ | 3-$NO_2$ | $(CH_2)_3N(CH_3)(CH_2)_3$ | 1 | 2-methoxyphenyl |
| $CH_3$ | 3-$NO_2$ | $(CH_2)_2N(CH_2C_6H_5)(CH_2)_2$ | 1 | phenyl |
| $CH_3$ | 3-$NO_2$ | $(CH_2)_2N(CH_2C_6H_5)(CH_2)_2$ | 1 | 2-cyanophenyl |
| $CH_3$ | 3-$NO_2$ | $(CH_2)_2NCH_2C_6H_5)(CH_2)_2$ | 1 | 2-methoxyphenyl |
| $CH_3$ | 3-$NO_2$ | phenylene-1,3-dimethylene | 1 | phenyl |
| $CH_3$ | 3-$NO_2$ | phenylene 1,3-dimethylene | 1 | 2-cyanophenyl |
| $CH_3$ | 3-$NO_2$ | phenylene 1,3-dimethylene | 1 | 2-methoxyphenyl |
| $CH_3$ | 3-$NO_2$ | phenylene 1,3-dimethylene | 1 | 1-naphthyl |
| $CH_3$ | 3-$NO_2$ | phenylene-1,4-dimethylene | 1 | phenyl |
| $CH_3$ | 3-$NO_2$ | phenylene-1,4-dimethylene | 1 | 2-cyanophenyl |
| $CH_3$ | 3-$NO_2$ | phenylene-1,4-dimethylene | 1 | 2-methoxyphenyl |
| $CH_3$ | 3-$NO_2$ | cyclohexylene-1,3-dimethylene | 1 | 2-methoxyphenyl |
| $CH_3$ | 3-$NO_2$ | $(CH_2)_5$ | 0 | phenyl |
| $CH_3$ | 3-$NO_2$ | $(CH_2)_5$ | 0 | 2,4-dimethylphenyl |
| $CH_3$ | 3-$NO_2$ | $(CH_2)_5$ | 0 | 3-methoxyphenyl |
| $CH_3$ | 3-$NO_2$ | cyclohexylene-1,3-dimethylene | 0 | 2-methoxyphenyl |
| $CH_3$ | 2-$NO_2$ | $(CH_2)_4$ | 1 | phenyl |
| $CH_3$ | 2-$NO_2$ | $(CH_2)_4$ | 1 | 2-nitrophenyl |
| $CH_3$ | 2-$NO_2$ | $(CH_2)_4$ | 1 | 2-cyanophenyl |

0194750

| $R^1$ | Ring A Substituent | Y | p | Ar |
|---|---|---|---|---|
| $CH_3$ | $2-NO_2$ | $(CH_2)_5$ | 1 | 2-tolyl |
| $CH_3$ | $2-NO_2$ | $(CH_2)_5$ | 1 | 2-methoxyphenyl |
| $CH_3$ | $2-NO_2$ | $(CH_2)_5$ | 1 | 1,4-benzodioxan-5-yl |
| $CH_3$ | $2-NO_2$ | $(CH_2)_5$ | 1 | 2-cyanophenyl |
| $CH_3$ | $2-NO_2$ | $(CH_2)_5$ | 1 | 1,4-benzodioxan-5-yl |
| $CH_3$ | 2-Cl | $(CH_2)_4$ | 1 | 2-cyanophenyl |
| $CH_3$ | 2-Cl | $(CH_2)_4$ | 1 | 2-methoxyphenyl |
| $CH_3$ | 2-Cl | $(CH_2)_4$ | 1 | 1,4-benzodioxan-5-yl |
| $CH_3$ | 2-Cl | $(CH_2)_5$ | 1 | 2-cyanophenyl |
| $CH_3$ | 2-Cl | $(CH_2)_5$ | 1 | 2-methoxyphenyl |
| $CH_3$ | 2-Cl | $(CH_2)_5$ | 1 | 2-acetylphenyl |
| $CH_3$ | 2-Cl | $(CH_2)_5$ | 1 | 1,4-benzdioxan-5-yl |
| $CH_3$ | 2-Cl | $(CH_2)_7$ | 1 | phenyl |
| $CH_3$ | 2-Cl | $(CH_2)_3O(CH_2)_4O(CH_2)_3$ | 1 | phenyl |
| $CH_3$ | 2-Cl | $(CH_2)_3O(CH_2)_4O(CH_2)_3$ | 1 | 2-cyanophenyl |
| $CH_3$ | 2-Cl | $(CH_2)_3O(CH_2)_4O(CH_2)_3$ | 1 | 2-methoxyphenyl |
| $CH_3$ | 2-Cl | $(CH_2)_3N(CH_3)(CH_2)_3$ | 1 | 2-cyanophenyl |
| $CH_3$ | 2-Cl | $(CH_2)_3N(CH_3)(CH_2)_3$ | 1 | 2-methoxyphenyl |
| $CH_3$ | 2-Cl | phenylene-1,3-dimethylene | 1 | phenyl |
| $CH_3$ | 2-Cl | phenylene-1,3-dimethylene | 1 | 2-cyanophenyl |
| $CH_3$ | 2-Cl | phenylene-1,3-dimethylene | 1 | 2-methoxyphenyl |
| $CH_3$ | 2-Cl | phenylene-1,2-diethylene | 1 | phenyl |
| $CH_3$ | 2-Cl | cyclohexylene-1,3-dimethylene | 1 | 2-cyanophenyl |
| $CH_3$ | 2-Cl | cyclohexylene-1,3-dimethylene | 1 | 2-methoxyphenyl |
| $CH_3$ | 2-Cl | phenylene-1,3-dimethylene | 0 | 3-methoxyphenyl |
| $CH_3$ | 2,3-diCl | $(CH_2)_5$ | 1 | 2-methoxyphenyl |
| $CH_3$ | 2,3-diCl | $(CH_2)_5O(CH_2)_5$ | 1 | 2-methoxyphenyl |
| $CH_3$ | 2,3-diCl | $(CH_2)_3O(CH_2)_4O(CH_2)_3$ | 1 | 2-methoxyphenyl |

| $R^1$ | Ring A Substituent | Y | p | Ar |
|---|---|---|---|---|
| $CH_3$ | 2-F-6-Cl | phenylene-1,3-dimethylene | 1 | phenyl |
| $CH_3$ | 2-F-6-Cl | phenylene-1,3-dimethylene | 1 | 2-cyanophenyl |
| $CH_3$ | 2-F-6-Cl | phenylene-1,3-dimethylene | 1 | 2-methoxyphenyl |
| $CH_3$ | 2-CN | $(CH_2)_4$ | 1 | 2-methoxyphenyl |
| $CH_3$ | 2-CN | $(CH_2)_5$ | 1 | 2-methoxyphenyl |
| $CH_3$ | 2-CN | $(CH_2)_3O(CH_2)_4O(CH_2)_3$ | 1 | 2-cyanophenyl |
| $CH_3$ | 2-CN | $(CH_2)_3O(CH_2)_4O(CH_2)_3$ | 1 | 2-methoxyphenyl |
| $CH_3$ | $2-CF_3$ | $(CH_2)_3$ | 1 | 2-cyanophenyl |
| $CH_3$ | $2-CF_3$ | $(CH_2)_4$ | 1 | 2-cyanophenyl |
| $CH_3$ | $2-CF_3$ | $(CH_2)_4$ | 1 | 2-tolyl |
| $CH_3$ | $2-CF_3$ | $(CH_2)_4$ | 1 | 2-methoxyphenyl |
| $CH_3$ | $2-CF_3$ | $(CH_2)_4$ | 1 | 1,4-benzodioxan-5-yl |
| $CH_3$ | $2-CF_3$ | $(CH_2)_6$ | 1 | 2-cyanophenyl |
| $CH_3$ | $2-CF_3$ | $(CH_2)_3O(CH_2)_4O(CH_2)_3$ | 1 | 2-methoxyphenyl |
| $CH_3$ | $2-CF_3$ | $(CH_2)_3O(CH_2)_4O(CH_2)_3$ | 1 | 1,4-benzodioxan-5-yl |
| $C_2H_5$ | $2-CF_3$ | $(CH_2)_3O(CH_2)_4O(CH_2)_3$ | 1 | 2-cyanophenyl |
| $CH_3$ | $2-CF_3$ | $(CH_2)_3O(CH_2)_4O(CH_2)_3$ | 1 | 2-cyanophenyl |
| $CH_3$ | $2-CH_3$ | $(CH_2)_5$ | 1 | 2-cyanophenyl |
| $CH_3$ | $2-CH_3$ | $(CH_2)_5$ | 1 | methoxyphenyl |
| $CH_3$ | $2-CH_3$ | $(CH_2)_6$ | 1 | phenyl |
| $CH_3$ | $2-CH_3$ | $(CH_2)_6$ | 1 | 2-cyanophenyl |
| $CH_3$ | $2-CH_3$ | $(CH_2)_6$ | 1 | 2-methoxyphenyl |
| $CH_3$ | $2-CH_3$ | phenylene-1,3-dimethylene | 1 | 2-cyanophenyl |
| $CH_3$ | $2-CH_3$ | phenylene-1,3-dimethylene | 1 | 2-methoxyphenyl |
| $CH_3$ | 2,3 =N-O-N= | $(CH_2)_4$ | 1 | 2-cyanophenyl |
| $CH_3$ | 2,3 =N-O-N= | $(CH_2)_4$ | 1 | 2-methoxyphenyl |
| $CH_3$ | 2,3 =N-O-N= | $(CH_2)_5$ | 1 | 2-methoxyphenyl |
| $CH_3$ | 2,3 =N-O-N= | $(CH_2)_3O(CH_2)_4O(CH_2)_3$ | 1 | 2-cyanophenyl |

The carboxylate starting materials for the above process were obtained by a similar process to that described in the second part of Example 3, using the appropriate 1,4-dihydro-5-alkoxycarbonyl-2,6-dimethyl-4-arylpyridine-3-carboxylic acid, hydroxybenzotriazole and the appropriate diamine. These are all known compounds apart from N-benzyl-N,N-bis-(2-aminoethyl)amine, which was obtained by the reaction of N,N-bis(2-aminoethyl)amine (21.6 g.) with phthalic anhydride (59.2 g.) in dimethylformamide (500 ml.) at 100°C. for 30 minutes, reaction of the N,N-bis(2-phthalimidoethyl)-amine (m.p. 181-182°C.) thus obtained (7.26 g.), benzylchloride (2.4 ml.), potassium carbonate (2.8 g.) and potassium iodide (3.36 g.) in dimethylformamide (100 ml.) at 120°C. for 4 hours, and then removal of the phthalyl protecting groups from the N-benzyl-N,N-bis-(2-phthalimidoethyl)amine (m.p. 134-136°C.) thus obtained (4.53 g.) with hydrazine hydrate (6 ml.) in ethanol (100 ml.) at laboratory temperature.

Example 5

A mixture of methyl 5-(N-5-aminopentylcarbamoyl)-4-o-chlorophenyl-1,4-dihydro-2,6-dimethylpyridine-3-carboxylate (Example 3; 2.02 g.), o-methoxyphenacyl bromide (0.57 g.) and dioxan (50 ml.) was stirred at laboratory temperature for 3 hours. Sodium borohydride (0.6 g.) was added in portions during 15 minutes and the mixture was stirred for a further 2 hours and then adjusted to pH 1 with concentrated aqueous hydrochloric acid. The mixture was evaporated to dryness and the residue was purified by flash chromatography on a silica gel (Merck 9385) column using an 80:20:3 v/v/v mixture of ethyl acetate, methanol and concentrated aqueous ammonia solution as eluent. There was thus obtained, as an oil, methyl 4-o-chlorophenyl-1,4-dihydro-5-N-[5-(2-hydroxy-2-o-methoxy-

phenylethylamino)pentyl]carbamoyl-2,6-dimethylpyridine-3-carboxylate hydrate, the structure of which was confirmed by elemental analysis and proton magnetic resonance spectroscopy as follows:-

Elemental analysis

Found:- C,62.5%; H,6.9%; N,7.2%. $C_{30}H_{38}N_{3}O_{5}Cl.H_{2}O$ requires C, 62.75%; H,7.0%; N,7.3%.

Proton magnetic resonance spectrum (in $CD_3SOCD_3+CD_3COOD$)

| Shift (δ) | Type | No of protons | Assignment |
|---|---|---|---|
| 0.95-1.6 | broad | 6 | $-C-CH_2-C-$ |
| 1.7-1.9 | 2 singlets | 6 | $-CH_3$ |
| 2.7-3.1 | complex | 6 | $NH-CH_2-$ |
| 3.4 | singlet | 3 | $CH_3OCO$ |
| 3.8 | singlet | 3 | $CH_3O$-phenyl |
| 5.05-5.15 | complex | 2 | pyridine 4H, -CHOH |
| 6.95-7.5 | complex | 9 | aromatic, pyridine NH |

The process described above was repeated using the appropriate amine and the appropriate phenacyl bromide as starting materials and there were thus obtained as oils the compounds described in the following table, the structures of all of which were confirmed by the above procedures:-

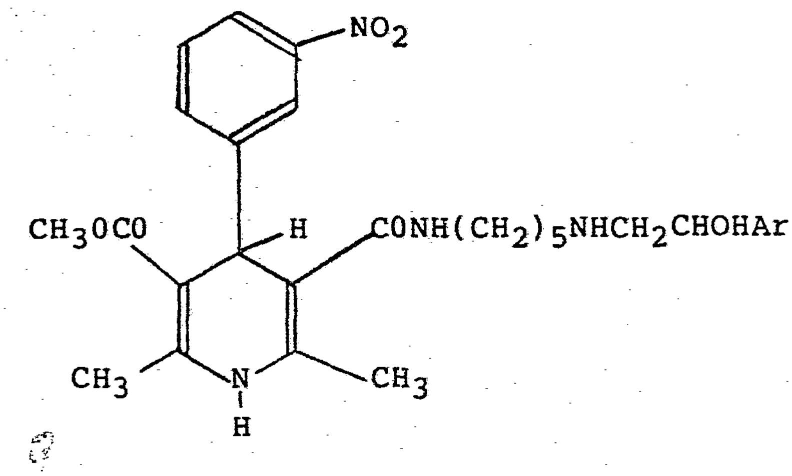

| Ar |
|---|
| 2-methoxyphenyl |
| 4-nitrophenyl |

Example 6

A solution of benzotriazol-1-yl 5-ethoxycarbonyl-1,4-dihydro-2,6-dimethyl-4-o-trifluoromethylpyridine-3-carboxylate (0.5 g.) and 1-(5-aminopentyl)amino-3-o-cyanophenoxypropan-2-ol (0.57 g.) in methylene chloride (20 ml.) was stirred at laboratory temperature for 20 hours, washed with water, dried and evaporated to dryness, and the residue was purified by flash chromatography on a silica gel (Merck 9385) column using as eluent a 60:35:20 v/v/v mixture of toluene, ethanol and ethyl acetate containing an increasing amount of concentrated aqueous ammonia solution, from 1% by volume to 2% by volume. There was thus obtained as an oil ethyl 5-N-[5-(3-o-cyanophenoxy-2-hydroxypropyl-amino)pentyl]carbamoyl-1,4-dihydro-2,6-dimethyl-4-o-trifluoromethylphenylpyridine-3-carboxylate, the structure of which was confirmed by elemental analysis,

0194750

mass spectroscopy and proton magnetic resonance spectroscopy.

The benzotriazolyl ester used as starting material was obtained as follows:-

A mixture of 1-hydroxybenzotriazole hydrate (3.3 g.), 3-ethoxycarbonyl-1,4-dihydro-2,6-dimethyl-4-o-trifluoromethylphenylpyridine-3-carboxylic acid (8.2 g.), dicyclohexylcarbodiimide (5.03 g.) and methylene chloride (160 ml.) was stirred at laboratory temperature for 2 hours and then filtered, and the filtrate was evaporated to dryness. The residue was stirred with diethyl ether and the mixture was filtered. There was thus obtained as solid product the desired ester, m.p. 153-157°C. (with decomposition).

The process described above was repeated using the appropriate benzotriazolyl ester and the appropriate amine as starting materials, and there were thus obtained as oils the compounds described in the following table, the structures of all of which were confirmed by the procedures described above:-

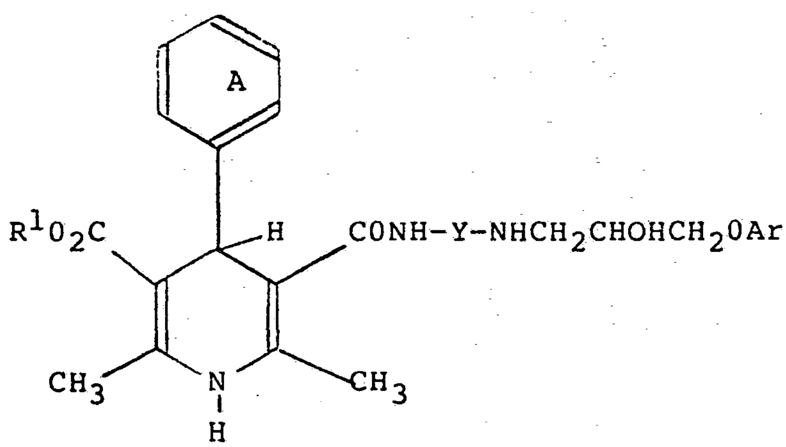

| $R^1$ | Ring A Substituent | Y | Ar |
|---|---|---|---|
| $CH_3$ | 3-$NO_2$ | $(CH_2)_2$ | phenyl |
| $CH_3$ | 2-CN | $(CH_2)_5$ | 2-cyanophenyl |
| $C_2H_5$ | 2-$CF_3$ | $(CH_2)_4$ | 2-cyanophenyl |
| $CH_3$ | 2-$CF_3$ | $(CH_2)_5$ | 2-cyanophenyl |
| $CH_3$ | 2,3 =N-O-N- | $(CH_2)_5$ | 2-cyanophenyl |

Example 7

A mixture of 2-(1,4-dihydro-5-methoxycarbonyl-2,6-dimethyl-4-*m*-nitrophenylpyridine-3-carboxamido)acetic acid (0.82 g.), dicyclohexylcarbodiimide (0.66 g.), 1-hydroxybenzotriazole hydrate (0.283 g.), 1-(2-aminoethyl)amino-3-phenoxypropan-2-ol (0.44 g.) and methylene chloride (20 ml.) was stirred at laboratory temperature for 24 hours, filtered and the filtrate was washed twice with 10% w/v aqueous sodium carbonate solution and once with water, dried and evaporated to dryness. The residue was partially purified by medium pressure liquid chromatography on a silica gel (Merck 9385) column using an 80:20:5 v/v/v mixture of ethyl acetate, methanol and concentrated aqueous ammonia solution as eluent, and was finally purified by preparative thick layer chromatography on silica gel plates (20 cm. x 20 cm., 20 mm. thick) using a 60:35:20:10 v/v/v/v mixture of toluene, ethanol, ethyl acetate and concentrated aqueous ammonia solution as eluent. There was thus obtained, as an oil, methyl 1,4-dihydro-5-*N*-{*N*-[2-(2-hydroxy-3-phenoxypropylamino)-ethyl]carbamoylmethyl} carbamoyl-2,6-dimethyl-4-*m*-nitrophenylpyridine-3-carboxylate, the structure of which was confirmed by elemental analysis, mass spectroscopy and proton magnetic resonance spectroscopy.

The acetic acid used as starting material was obtained by the reaction of 5-ethoxycarbonyl-1,4-dihydro-2,6-dimethyl-4-*m*-nitrophenylpyridine-3-carboxylic acid (1.25 g.), dicyclohexylcarbodiimide (0.74 g.), 1-hydroxybenzotriazole hydrate (0.49 g.), ethyl glycinate hydrochloride (0.51 g.) and triethylamine (0.36 g.) in methylene chloride (30 ml.) at laboratory temperature for 24 hours, followed by hydrolysis of the ethyl ester thus obtained (1.4 g.) with aqueous N-sodium hydroxide solution (33 ml.) and ethanol (4 ml.) at laboratory temperature for 18 hours.

The process described above was repeated using the appropriate alkanoic acid and the appropriate 1-(2-aminoethylamino)-3-aryloxypropan-2-ol as starting materials and there were thus obtained as oils the compounds described in the following table, the structures of all of which were confirmed by the procedures described above.

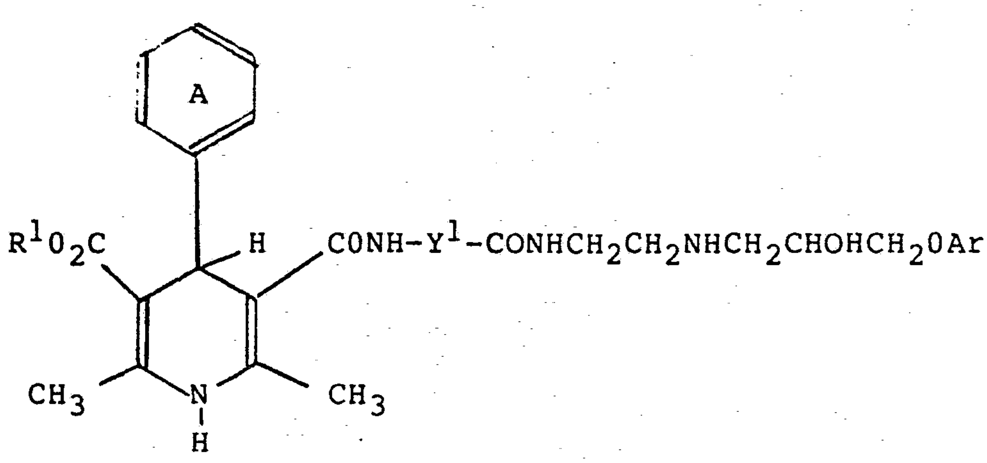

| $R^1$ | Ring A Substituent | $Y^1$ | Ar |
|---|---|---|---|
| $CH_3$ | 3-$NO_2$ | $CH_2$ | 2-cyanophenyl |
| $CH_3$ | 3-$NO_2$ | $CH_2$ | 2-naphthyl |
| $CH_3$ | 3-$NO_2$ | $CH_2$ | 1,2,3,4-tetrahydro-naphth-5-yl |
| $CH_3$ | 3-$NO_2$ | $CH_2CH_2$ | phenyl |
| $CH_3$ | 3-$NO_2$ | $CH_2CH_2$ | 2-naphthyl |
| $CH_3$ | 3-$NO_2$ | $C(CH_3)_2$ | 2-naphthyl |
| $CH_3$ | 2-Cl | $CH_2$ | phenyl |
| $CH_3$ | 2-Cl | $CH_2$ | 2-cyanophenyl |
| $CH_3$ | 2-Cl | $CH_2$ | 2-naphthyl |
| $C_2H_5$ | 2-$CF_3$ | $CH_2$ | 2-cyanophenyl |

Example 8

An intimate mixture of 1-amino-3-phenoxypropan-2-ol (0.31 g.) and 2-[3-(2-bromoethyl)phenyl]ethyl 1,4-dihydro-5-methoxycarbonyl-2,6-dimethyl-4-*m*-nitrophenylpyridine-3-carboxylate (1.0 g.) was melted and heated at 100°C. for 16 hours, cooled and stirred with methylene chloride (20 ml.), and the mixture was filtered. The filtrate was evaporated to dryness and the residue was purified by flash chromatography on a silica gel (Merck 9385) column using a 90:10:3 v/v/v mixture of ethyl acetate, methanol and concentrated aqueous ammonia solution as eluent. There was thus obtained, as an oil, 2-{3-(2-hydroxy-3-phenoxypropylamino)ethyl]phenyl}ethyl 1,4-dihydro-5-methoxycarbonyl-2,6-dimethyl-4-*m*-nitrophenylpyridine-3-carboxylate hydrate, the structure of which was confirmed by elemental analysis and proton magnetic resonance spectroscopy as follows:-

Elemental analysis

Found:- C, 65.8%; H,6.3%; N,6.5%.$C_{35}H_{39}N_3O_8.H_2O$ requires C, 65.8%; H,6.3%; N,6.6%.

Proton magnetic resonance spectrum (in $CDCl_3$)

| Shift (δ) | Type | No of Protons | Assignment |
|---|---|---|---|
| 2.1-2.5 | complex | 10 | $CH_3$,phenyl-$CH_2$- |
| 2.6-3.0 | complex | 6 | N-$CH_2$, OH, NH |
| 3.65 | singlet | 3 | CH3OCO |
| 3.95-4.05 | single + complex | 3 | -O<u>CH</u>$_2$C<u>H</u>OH- |
| 4.2-4.4 | triplet | 2 | $COOCH_2$- |
| 5.05 | singlet | 1 | pyridine 4H |
| 5.9 | singlet | 1 | pyridine NH |
| 6.9-7.6 | complex | 11 | aromatic |
| 7.95-8.05 | complex | 2 | aromatic |

The dihydropyridine starting material was obtained by the reaction of 1,3-di-(2-hydroxyethyl)-benzene (5.4 g.) and 48% aqueous hydrobromic acid (160 ml.) at 80°C. for 16 hours, with continuous toluene extraction of the product, followed by reaction of the 2-[3-(2-bromoethyl)phenyl]ethanol thus obtained (4.6 g.) with benzotriazol-1-yl 1,4-dihydro-5-methoxycarbonyl-2,6-dimethyl-4-m-nitrophenylpyridine-3-carboxylate (Example 3; 6.0 g.) at 100°C. for 16 hours;

The process described in the first paragraph above was repeated using the appropriate dihydropyridine ester and the appropriate 1-amino-3-aryloxypropan-2-ol as starting materials, and there were thus obtained as oils the compounds described in the following table, the

structures of all of which were confirmed by the procedures stated above:-

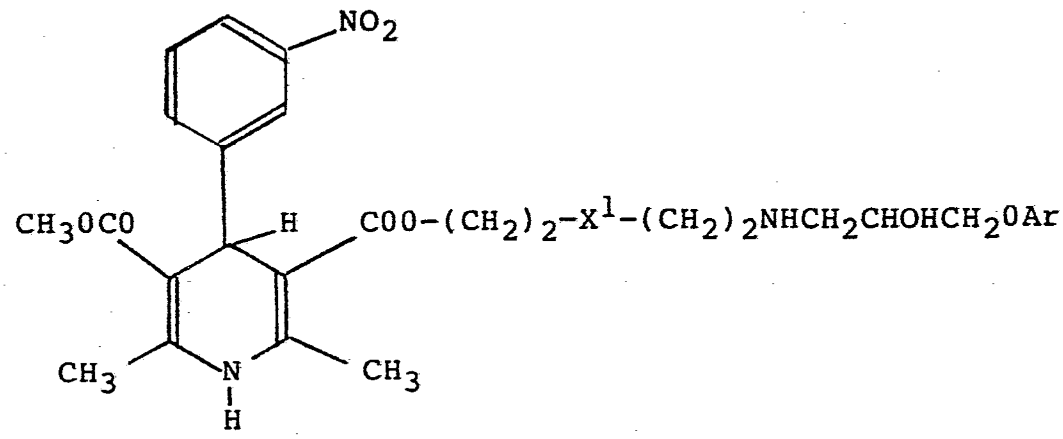

| $X^1$ | Ar |
|---|---|
| 1,3-phenylene | 2-cyanophenyl |
| 1,3-phenylene | 2-methoxyphenyl |
| -O- | phenyl |
| -O- | 2-cyanophenyl |

The process described in the first paragraph above was repeated using the appropriate haloalkyl dihydropyridine-3-carboxylate and the appropriate 1-(omega-aminoethyl)amino-3-aryloxypropan-2-ol as starting materials, and there were thus obtained the compounds described in the following table, many of which were characterised as oxalate salts formed by reacting the free base with an ethereal oxalic acid solution:-

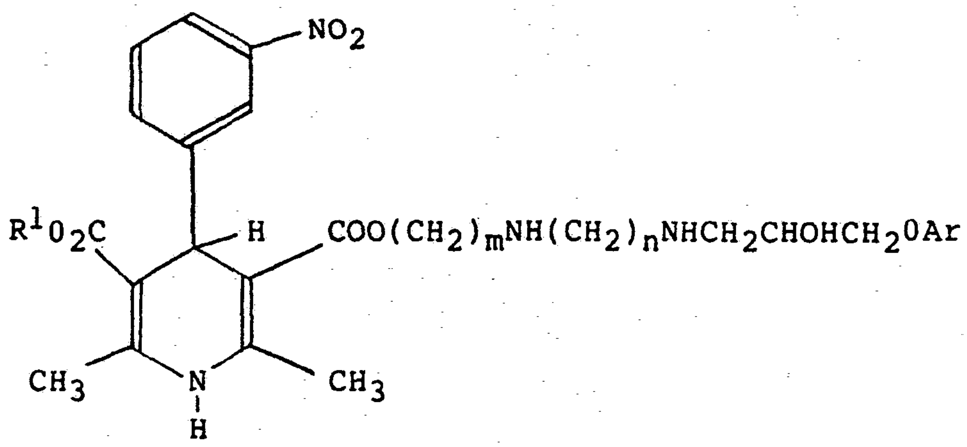

| $R^1$ | m | n | Ar | m.p. (°C.) of oxalate salt |
|---|---|---|---|---|
| $C_2H_5$ | 2 | 2 | 1-naphthyl | 219-220 |
| $C_2H_5$ | 3 | 2 | phenyl | 238-239 |
| $CH_3$ | 3 | 2 | 2-cyanophenyl | 236-238 |
| $C_2H_5$ | 3 | 2 | 2-cyanophenyl | 235-236 |
| $CH_3$ | 3 | 2 | 2-fluorophenyl | - |
| $C_2H_5$ | 3 | 2 | 2,3-dichlorophenyl | 229-230 |
| $C_2H_5$ | 3 | 2 | 2-tolyl | 236 |
| $C_2H_5$ | 3 | 2 | 1-naphthyl | 230-231 |
| $C_2H_5$ | 3 | 4 | 1-naphthyl | 183-185 |

Example 9

An intimate mixture of 1-amino-3-*o*-cyanophenoxypropan-2-ol (1.15 g.) and 6-chlorohexyl 1,4-dihydro-5-methoxycarbonyl-2,6-dimethyl-4-*m*-nitrophenylpyridine-3-carboxylate (1.35 g.)

was melted and heated at 100°C. for 4 hours and then stirred with methylene chloride (20 ml.), and the mixture was filtered. The filtrate was evaporated to dryness and the residue was purified by flash chromatography on a silica gel (Merck 9385) column using a 9:1 v/v mixture of methylene chloride and methanol as eluent. There was thus obtained as an oil 6-(3-o-cyanophenoxy-2-hydroxypropylamino)hexyl 1,4-dihydro-5-methoxycarbonyl-2,6-dimethyl-4-m-nitrophenylpyridine-3-carboxylate, the structure of which was confirmed by proton magnetic resonance spectrocopy in $CD_3SOCD_3$ as follows:-

| Shift (δ) | Type | No. of protons | Assignment |
|---|---|---|---|
| 1.00-1.90 | multiplet | 8 | $C-(CH_2)-C$ |
| 2.31 | singlet | 6 | $CH_3$ |
| 2.60-3.00 | multiplet | 4 | $C-(CH_2)-N$ |
| 3.58 | singlet | 3 | $CH_3O$ |
| 3.80-4.20 | multiplet | 5 | $C-CH_2O$, CHOH |
| 5.00 | singlet | 1 | pyridine 4H |
| 7.00-8.05 | multiplet | 8 | aromatic |
| 9.04 | singlet | 1 | pyridine NH |

The chlorohexyl pyridine-3-carboxylate used as starting material was obtained as follows:-

Diketene (28.8 ml.) was added dropwise to stirred 6-chlorohexanol which was heated to 70°C., and the mixture was stirred at 75°C. for 3 hours and then distilled under reduced pressure. There was thus obtained 6-chlorohexyl acetoacetate, b.p. 100-102°C/0.05 mm.Hg.

A mixture of the above ester (16.5 g.), m-nitrobenzaldehyde (11.3 g.), methyl aminocrotonate (8.625 g.) and isopropanol (100 ml.) was heated under

reflux for 4 hours and then evaporated to dryness under reduced pressure. The residue was purified by flash chromatography on a silica gel (Merck 9385, 300 g.) column using methylene chloride as eluent. There was thus obtained as a yellow solid 6-chlorohexyl 1,4-dihydro-5-methoxycarbonyl-2,6-dimethyl-4-m-nitrophenylpyridine-3-carboxylate.

The process described above was repeated using the appropriate haloalkyl 1,4-dihydropyridine-3-carboxylate and the appropriate 1-amino-3-aryloxypropan-2-ol as starting materials and there were thus obtained as oils the compounds described in the following table, the structures of all of which were confirmed by elemental analysis and proton magnetic resonance:-

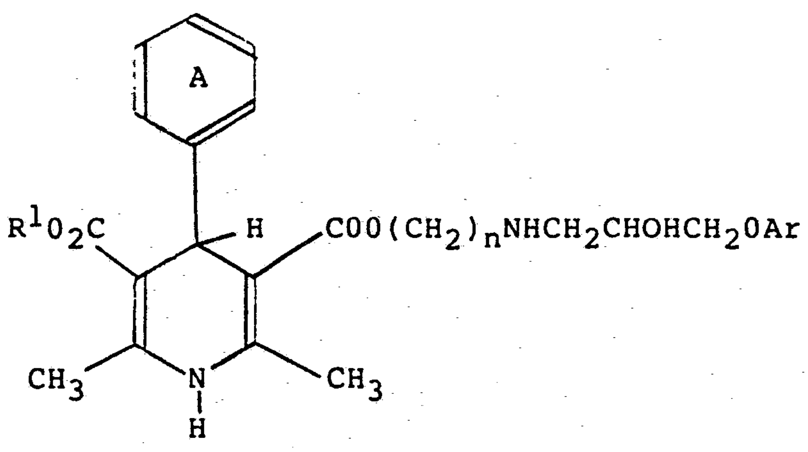

| $R^1$ | Ring A Substituent | n | Ar |
|---|---|---|---|
| $CH_3$ | 3-$NO_2$ | 3 | 2-cyanophenyl |
| $C_2H_5$ | 3-$NO_2$ | 3 | 2-cyanophenyl |
| $CH_3$ | 3-$NO_2$ | 3 | 2-tolyl |
| $C_2H_5$ | 3-$NO_2$ | 3 | 2-tolyl |
| $CH_3$ | 3-$NO_2$ | 4 | 2-cyanophenyl |
| $C_2H_5$ | 3-$NO_2$ | 4 | 2-tolyl |
| $CH_3$ | 3-$NO_2$ | 5 | 2-chlorophenyl |
| $CH_3$ | 3-$NO_2$ | 5 | 2-cyanophenyl |
| $CH_3$ | 3-$NO_2$ | 6 | phenyl |
| $CH_3$ | 3-$NO_2$ | 6 | 2-chlorophenyl |
| $CH_3$ | 3-$NO_2$ | 6 | 2-tolyl |
| $CH_3$ | 3-$NO_2$ | 6 | 2-methoxyphenyl |
| $CH_3$ | 3-$NO_2$ | 6 | 2-ethoxyphenyl |
| $CH_3$ | 3-$NO_2$ | 7 | phenyl |
| $CH_3$ | 3-$NO_2$ | 7 | 2-cyanophenyl |
| $CH_3$ | 3-$NO_2$ | 7 | 2-methoxyphenyl |
| $CH_3$ | 3-$NO_2$ | 11 | 2-cyanophenyl |
| $CH_3$ | 2-Cl | 4 | phenyl |
| $CH_3$ | 2-Cl | 5 | phenyl |
| $CH_3$ | 2-Cl | 5 | 2-cyanophenyl |
| $CH_3$ | 2-Cl | 5 | 2-methoxyphenyl |
| $CH_3$ | 2-Cl | 6 | phenyl |
| $CH_3$ | 2-Cl | 6 | 2-cyanophenyl |
| $CH_3$ | 2-Cl | 6 | 2-methoxyphenyl |
| $CH_3$ | 2-$CH_3$ | 6 | phenyl |

Example 10

A mixture of 2-[4-(2-aminoethyl)piperazin-1-yl]ethyl 1,4-dihydro-5-methoxycarbonyl-2,6-dimethyl-4-m-nitrophenylpyridine-3-carboxylate (1.6 g.), 1-o-cyanophenoxy-2,3-epoxypropane (0.57 g.) and isopropanol (20 ml.) was heated under reflux for 18 hours, cooled and evaporated to dryness and the residue was purified by flash chromatography on a silica gel (Merck 9385) column using an 80:17:3 v/v/v mixture of ethyl acetate, methanol and aqueous ammonia solution (specific gravity 0.88) as eluent. There was thus obtained as an oil 2-{4-[2-(3-o-cyanophenoxy-2-hydroxypropylamino)ethyl]-piperazin-1-yl}ethyl 1,4-dihydro-5-methoxycarbonyl-2,6-dimethyl-4-m-nitrophenylpyridine-3-carboxylate hydrate, the structure of which was confirmed by elemental analysis and proton magentic resonance spectroscopy as follows:-

Elemental analysis

Found:- C,59.6%; H,6.4%; N,12.1%. $C_{34}H_{42}H_6O_8.H_2O$ requires C, 59.98%; H,6.5%; N,12.3%.

Proton magnetic resonance spectrum (in $CD_3SOCD_3$)

| Shift (δ) | Type | No of Protons | Assignment |
|---|---|---|---|
| 2.25-2.5 | complex | 18 | $-CH_3-$, N-CH2- |
| 2.6-2.75 | complex | 4 | $-NHCH_2-$, $NCH_2CH_2O-$ |
| 3.25-3.5 | broad | 2 | OH, NH |
| 3.55 | singlet | 3 | $CH_3O$ |
| 3.9 | quintet | 1 | CHOH |
| 3.85-4.15 | complex | 4 | $CH_2O$ |
| 5.0 | singlet | 1 | pyridine 4H |
| 7.05-8.05 | complex | 8 | aromatic |
| 9.05 | singlet | 1 | pyridine NH |

The dihydropyridine used as starting material was obtained by the reaction of N-2-hydroxyethylpiperazine (6.5 g.), N-2-bromoethylphthalimide (12.0 g.) and potassium carbonate (7.0 g.) in dimethoxyethane (200 ml.) at laboratory temperature for 16 hours, the reaction of the 1-(2-hydroxyethyl)-4-(2-phthalimidoethyl)piperazine thus obtained (2.5 g.) and benzotriazol-1-yl 1,4-dihydro-5-methoxycarbonyl-2,6-dimethyl-4-m-nitrophenylpyridine-3-carboxylate (3.7 g.) in dimethoxyethane (50 ml.) at 90°C. for 16 hours and the removal of the phthalyl protecting group by reaction with hydrazine hydrate in ethanol solution at laboratory temperature.

The process described in the first paragraph above was repeated using the appropriate amine and the appropriate epoxide as starting materials, and there were thus obtained as oils the compounds described in the following table, the structures of which were confirmed by elemental analysis and proton magnetic resonance spectroscopy:-

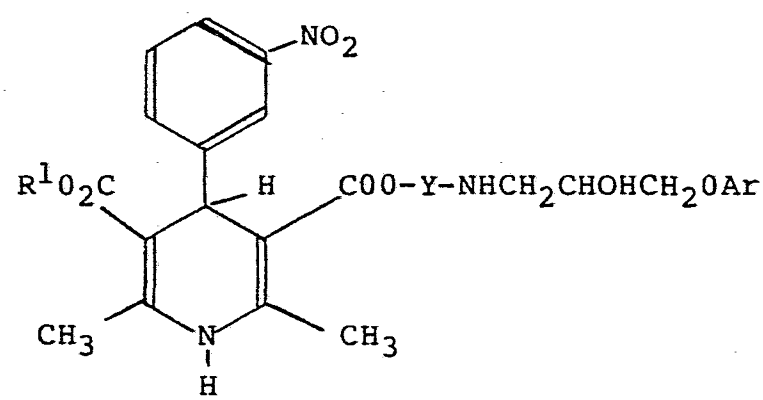

| $R^1$ | Y | Ar |
|---|---|---|
| $C_2H_5$ | $(CH_2)_3$ | 2-nitrophenyl |
| $C_2H_5$ | $(CH_2)_3$ | 1-naphthyl |
| $CH_3$ | $(CH_2)_5$ | 2-methoxyphenyl |

What we claim is:-

1. A dihydropyridine of the formula:-

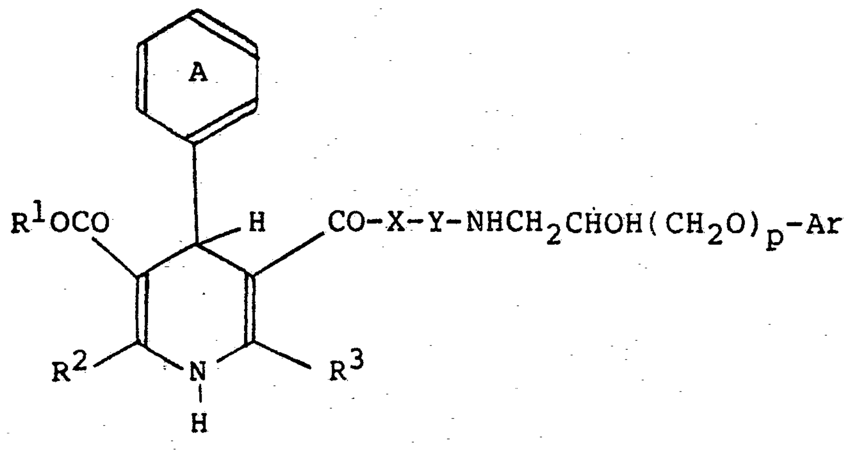

wherein $R^1$ is alkyl or alkoxyalkyl each of up to 6 carbon atoms: wherein $R^2$ and $R^3$, which may be the same or different, each is alkyl of up to 6 carbon atoms; wherein benzene ring A bears one or more substituents selected from halogeno, cyano, nitro, trifluoromethyl and alkyl of up to 6 carbon atoms or bears the substituent

=N-O-N=

attached to the 2- and 3-positions (that is, to form a benzo-2,1,3-oxadiazole nucleus); wherein Ar is phenyl, naphthyl, tetrahydronaphthyl, indanyl or indenyl which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms; or Ar is a 5- or 6-membered heterocyclic ring containing 1, 2 or 3 heteroatoms selected from oxygen, nitrogen and

sulphur, which ring is saturated or unsaturated, which ring is unsubstituted or bears one or more substituents selected from oxo, halogeno, trifluoromethyl, phenyl and morpholino, and alkyl and alkoxy each of up to 6 carbon atoms, and arylalkyl of up to 12 carbon atoms, and which ring may also be fused to a benzene ring, Ar being joined to the rest of the molecule either from the heterocyclic ring or from the fused benzene ring;

wherein p is 0 or 1;

wherein X is -O- or -NH-;

and wherein Y is straight-or branched-chain alkylene of 2 to 12 carbon atoms which may optionally be interrupted by one or two groups selected from oxygen (-O-), sulphur (-S-), imino and substituted imino ($-NR^4$ wherein $R^4$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;

or an acid-addition salt thereof.

2. A dihydropyridine as claimed in claim 1 wherein $R^1$ is alkyl or alkoxyalkyl each of up to 4 carbon atoms, wherein $R^2$ and $R^3$ are both methyl, wherein benzene ring A bears one or two substituents selected from fluoro and chloro, or bears one cyano, nitro, trifluoromethyl or methyl substituent, or the 2,3 =N-O-N= substituent, wherein Ar is phenyl which is unsubstituted or which bears one or two chloro or methyl substituents, or one fluoro, chloro, nitro, carbamoyl, cyano, methyl, methoxy, ethoxy, methoxyethoxy, acetyl, carbamoylmethyl or acetamido substituent, or Ar is unsubstituted naphthyl or tetrahydronaphthyl, or Ar is 1,4-benzodioxan-5-yl, 4-morpholino-1,2,5-thiadiazol-3-yl, 4-indolyl or 4-oxochroman-8-yl;

wherein p is 0 or 1, wherein X is -NH- and wherein Y is straight-chain alkylene of 2 to 12 carbon atoms, or branched-chain alkylene of 2 to 6 carbon atoms, or straight-chain alkylene of 2 to 12 carbon atoms which is interrupted by one oxygen, imino, alkylimino, aralkylamino, phenylene, cyclohexylene or amido group, or which is interrupted by two oxygen groups, or an acid-addition salt thereof.

3. A dihydropyridine as claimed in claim 1 wherein $R^1$, $R^2$, $R^3$, ring A and Ar have the meanings stated in claim 2, wherein p is 1, wherein X is -O- and wherein Y is straight-chain alkylene of 2 to 12 carbon atoms which is interrupted by one imino, alkylimino, aralkylimino, phenylene, cyclohexylene or amido group, or an acid-addition salt thereof.

4. A dihydropyridine as claimed in claim 2 or 3 wherein $R^1$ is methyl or ethyl, ring A bears a 2- or 3-nitro or a 2-fluoro, 2-chloro, 2-trifluoromethyl or 2-methyl substituent, Ar is phenyl which is unsubstituted or bears a 2-chloro, 2-cyano or 2-methoxy substituent, or Ar is unsubstituted naphthyl or 1,4-benzodioxan-5-yl, and p is 1.

5. The compounds :-

methyl 1,4-dihydro-2,6-dimethyl-4-m-nitrophenyl-5-[3-{N-(2-[2-hydroxy-3-phenoxypropylamino]ethyl)carbamoyl}-propoxycarbonyl]pyridine-3-carboxylate and

methyl 5-N-[5-(3-o-cyanophenoxy-2-hydroxypropylamino)-pentyl]carbamoyl-1,4-dihydro-2,6-dimethyl-4-m-nitrophenylpyridine-3-carboxylate.

6. The compounds:-

methyl 4-o-chlorophenyl-1,4-dihydro-5-N-[5-(2-hydroxy-3-o-methoxyphenoxypropylamino)pentyl]carbamoyl-2,6-dimethylpyridine-3-carboxylate;

methyl 1,4-dihydro-5-N-[5-(2-hydroxy-2-o-methoxyphenyl-ethylamino)pentyl]carbamoyl-2,6-dimethyl-4-m-

nitrophenylpyridine-3-carboxylate;
methyl 4-o-chlorophenyl-1,4-dihydro-5-N-[3-(2-hydroxy-3-phenoxypropylaminomethyl)benzyl]carbamoyl-2,6-dimethylpyridine-3-carboxylate and
methyl 4-(2-chloro-6-fluorophenyl)-5-N-[3-(3-o-cyanophenoxy-2-hydroxypropylaminomethyl)benzyl]carbamoyl-1,4-dihydro-2,6-dimethylpyridine-3-carboxylate.

7. The compounds:-
2-{3-[2-(2-hydroxy-3-phenoxypropylamino)ethyl]phenyl}-ethyl 4-o-chlorophenyl-1,4-dihydro-5-methoxycarbonyl-2,6-dimethylpyridine-3-carboxylate and
4-[N-5-(3-o-cyanophenoxy-2-hydroxypropylamino)pentylcarbamoyl]-butyl 1,4-dihydro-5-methoxycarbonyl-2,6-dimethyl-4-m-nitrophenylpyridine-3-carboxylate.

8. A process for the manufacture of a dihydropyridine, claimed in any of claims 1 to 7, which comprises:-
(a) the reaction of an amine of the formula:-

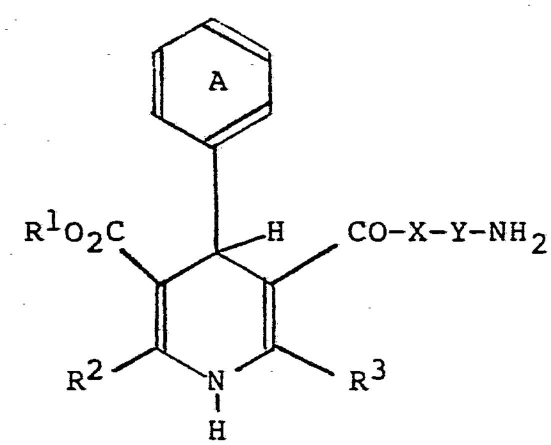

wherein A, $R^1$, $R^2$, $R^3$, X and Y have the meanings stated in any of claims 1 to 4, with an epoxide of the formula:-

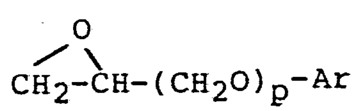

wherein Ar and p have the meanings stated in any of claims 1 to 4, or, when p is 0, with a haloketone of the formula

$$HalCH_2CO\text{-}Ar$$

wherein Ar has the meaning stated above and where Hal stands for a halogeno group, followed by reduction of the aminoketone thus obtained; or

(b) for the manufacture of a dihydropyridine wherein the group -Y-is alkylene interrupted by an amido group -CONH-, the reaction of an acid of the formula:-

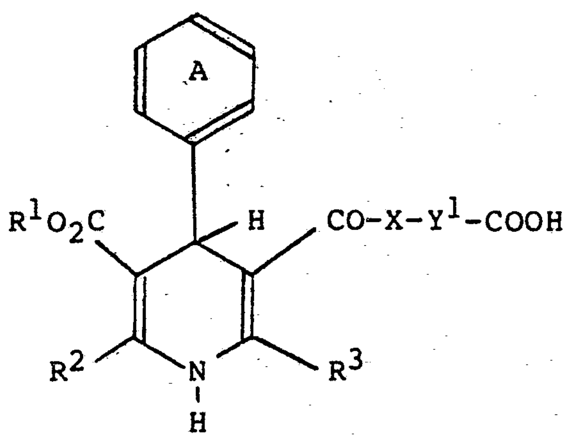

wherein A, $R^1$, $R^2$, $R^3$ and X have the meanings stated above, or an activated derivative thereof, with an amine of the formula:-

$$H_2N\text{-}Y^2\text{-}NHCH_2CHOH(CH_2O)_pAr$$

wherein Ar and p have the meanings stated above, and wherein $Y^1$ and $Y^2$ are such that $-Y^1\text{-}CONH\text{-}Y^2-$ has the same meaning as stated above for Y; or

(c) the reaction of an acid of the formula:-

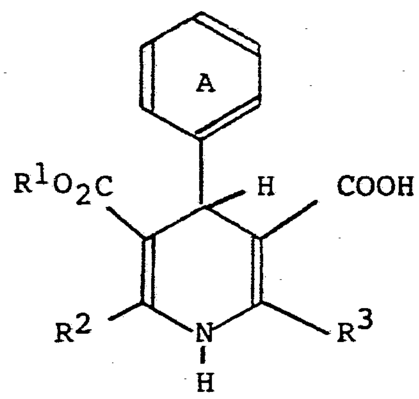

wherein A, $R^1$, $R^2$ and $R^3$ have the meanings stated above, or an activated derivative thereof, with an alcohol or amine of the formula:-

$$H-X-Y-NHCH_2CHOH(CH_2O)_p-Ar$$

wherein Ar, p, X and Y have the meanings stated above; or the reaction of a compound of the formula:-

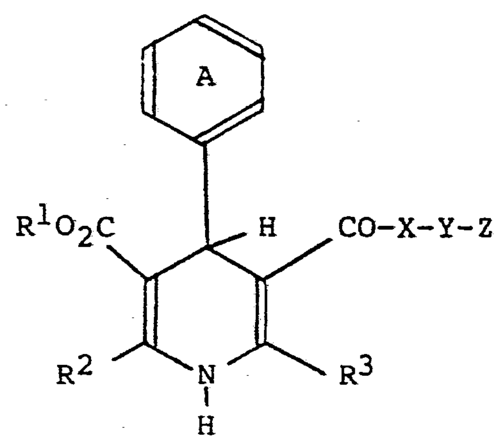

wherein A, $R^1$, $R^2$, $R^3$, X and Y have the meanings stated above and wherein Z stands for a displaceable group, with an amine of the formula:-

$$H_2NCH_2CHOH(CH_2O)_p-Ar$$

wherein Ar and p have the meanings stated above; or

(e) the reaction of an aldehyde of the formula

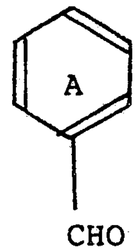

wherein A has the meaning stated above, an aminocrotonate of the formula

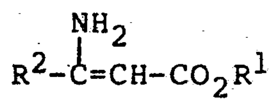

wherein $R^1$ and $R^2$ have the meanings stated above and a ketoacid derivative of the formula

$$R^3COCH_2CO-X-Y-NHCH_2CHOH(CH_2O)_p-Ar$$

wherein Ar, p, $R^3$, X and Y have the meanings stated above.

9. A pharmaceutical composition comprising as active ingredient at least one dihydropyridine, claimed in any of claims 1 to 7, or an acid-addition salt thereof, in association with a pharmaceutically-acceptable diluent or carrier therefor.

10. The use of a compound, claimed in any of claims 1 to 7, for the manufacture of a medicament for producing an antihypertensive effect in a warm-blooded animal.

REGINALD PETER SLATCHER

AUTHORISED REPRESENTATIVE
General Authorization No. 97

PS33364
SE07
RPS/MJW : 04FEB86

Claims for Austria

What we claim is:-

1. A process for the manufacture of a dihydropyridine of the formula:-

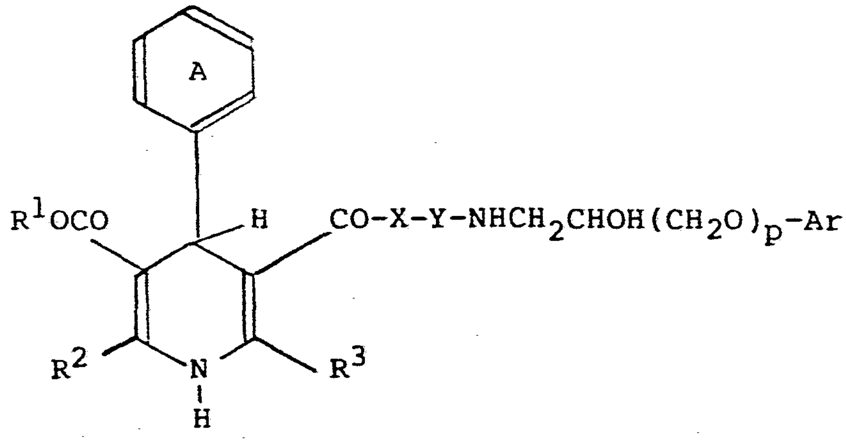

wherein $R^1$ is alkyl or alkoxyalkyl each of up to 6 carbon atoms:
wherein $R^2$ and $R^3$, which may be the same or different, each is alkyl of up to 6 carbon atoms;
wherein benzene ring A bears one or more substituents selected from halogeno, cyano, nitro, trifluoromethyl and alkyl of up to 6 carbon atoms or bears the substituent

=N-O-N=

attached to the 2- and 3-positions (that is, to form a benzo-2,1,3-oxadiazole nucleus);
wherein Ar is phenyl, naphthyl, tetrahydronaphthyl, indanyl or indenyl which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
or Ar is a 5- or 6-membered heterocyclic ring containing 1, 2 or 3 heteroatoms selected from oxygen, nitrogen and

sulphur, which ring is saturated or unsaturated, which ring is unsubstituted or bears one or more substituents selected from oxo, halogeno, trifluoromethyl, phenyl and morpholino, and alkyl and alkoxy each of up to 6 carbon atoms, and arylalkyl of up to 12 carbon atoms, and which ring may also be fused to a benzene ring, Ar being joined to the rest of the molecule either from the heterocyclic ring or from the fused benzene ring;
wherein p is 0 or 1;
wherein X is -O- or -NH-;
and wherein Y is straight-or branched-chain alkylene of 2 to 12 carbon atoms which may optionally be interrupted by one or two groups selected from oxygen (-O-), sulphur (-S-), imino and substituted imino ($-NR^4$ wherein $R^4$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;
or an acid-addition salt thereof, characterised by:-

(a) the reaction of an amine of the formula:-

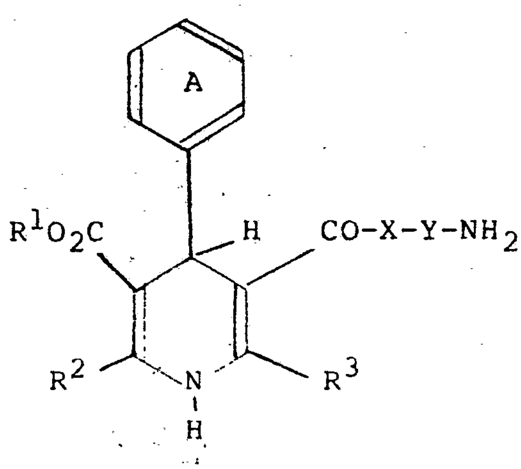

wherein A, $R^1$, $R^2$, $R^3$, X and Y have the meanings stated above, with an epoxide of the formula:-

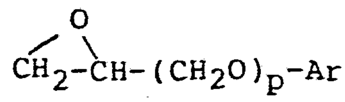

wherein Ar and p have the meanings stated above, or, when p is 0, with a haloketone of the formula

$$HalCH_2CO\text{-}Ar$$

wherein Ar has the meaning stated above and where Hal stands for a halogeno group, followed by reduction of the aminoketone thus obtained; or

(b) for the manufacture of a dihydropyridine wherein the group -Y-is alkylene interrupted by an amido group -CONH-, the reaction of an acid of the formula:-

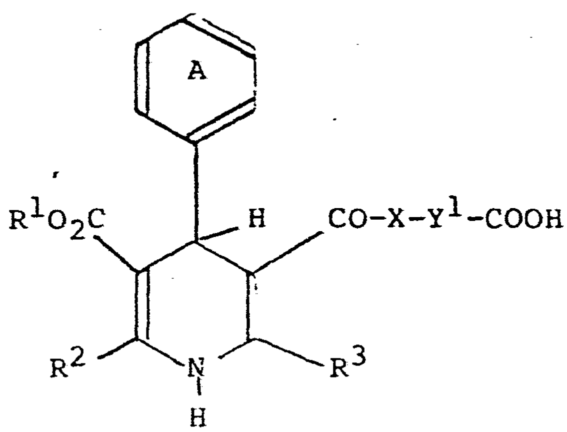

wherein A, $R^1$, $R^2$, $R^3$ and X have the meanings stated above, or an activated derivative thereof, with an amine of the formula:-

$$H_2N-Y^2-NHCH_2CHOH(CH_2O)_pAr$$

wherein Ar and p have the meanings stated above, and wherein $Y^1$ and $Y^2$ are such that $-Y^1-CONH-Y^2-$ has the same meaning as stated above for Y; or

(c) the reaction of an acid of the formula:-

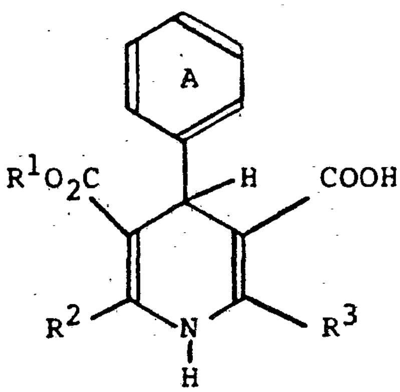

wherein A, $R^1$, $R^2$ and $R^3$ have the meanings stated above, or an activated derivative thereof, with an alcohol or amine of the formula:-

$$H-X-Y-NHCH_2CHOH(CH_2O)_p-Ar$$

wherein Ar, p, X and Y have the meanings stated above; or

the reaction of a compound of the formula:-

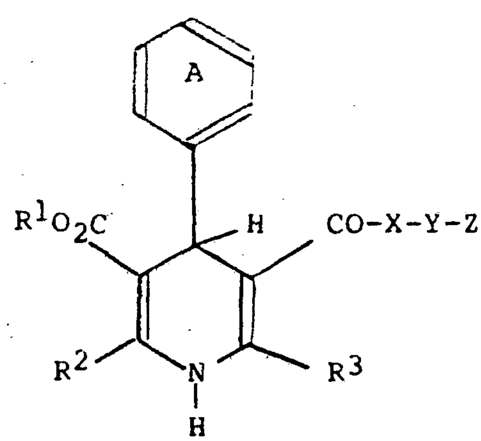

 0194750

wherein A, $R^1$, $R^2$, $R^3$, X and Y have the meanings stated above and wherein Z stands for a displaceable group, with an amine of the formula:-

$$H_2NCH_2CHOH(CH_2O)_p\text{-}Ar$$

wherein Ar and p have the meanings stated above; or

(e) the reaction of an aldehyde of the formula

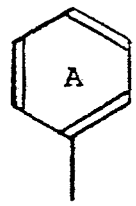

CHO

wherein A has the meaning stated above, an aminocrotonate of the formula

$$R^2\text{-}C(NH_2)\text{=}CH\text{-}CO_2R^1$$

wherein $R^1$ and $R^2$ have the meanings stated above and a ketoacid derivative of the formula

$$R^3COCH_2CO\text{-}X\text{-}Y\text{-}NHCH_2CHOH(CH_2O)_p\text{-}Ar$$

wherein Ar, p, $R^3$, X and Y have the meanings stated above.

2. A process as claimed in claim 1 wherein in the starting materials $R^1$ is alkyl or alkoxyalkyl each of up to 4 carbon atoms, $R^2$ and $R^3$ are both methyl, benzene ring A bears one or two substituents selected from fluoro and chloro, or

bears one cyano, nitro, trifluoromethyl or methyl substituent, or the 2,3 =N-0-N= substituent, Ar is phenyl which is unsubstituted or which bears one or two chloro or methyl substituents, or one fluoro, chloro, nitro, carbamoyl, cyano, methyl, methoxy, ethoxy, methoxyethoxy, acetyl, carbamoylmethyl or acetamido substituent, or Ar is unsubstituted naphthyl or tetrahydronaphthyl, or Ar is 1,4-benzodioxan-5-yl, 4-morpholino-1,2,5-thiadiazol-3-yl, 4-indolyl or 4-oxochroman-8-yl;

p is 0 or 1, X is -NH- and Y is straight-chain alkylene of 2 to 12 carbon atoms, or branched-chain alkylene of 2 to 6 carbon atoms, or straight-chain alkylene of 2 to 12 carbon atoms which is interrupted by one oxygen, imino, alkylimino, aralkylamino, phenylene, cyclohexylene or amido group, or which is interrupted by two oxygen groups.

3. A process as claimed in claim 1 wherein in the starting materials $R^1$, $R^2$, $R^3$, ring A and Ar have the meanings stated in claim 2, p is 1, X is -0- and Y is straight-chain alkylene of 2 to 12 carbon atoms which is interrupted by one imino, alkylimino, aralkylimino, phenylene, cyclohexylene or amido group.

4. A process as claimed in claim 2 or 3 wherein in the starting materials $R^1$ is methyl or ethyl, ring A bears a 2- or 3-nitro or a 2-fluoro, 2-chloro, 2-trifluoromethyl or 2-methyl substituent, Ar is phenyl which is unsubstituted or bears a 2-chloro, 2-cyano or 2-methoxy substituent, or Ar is unsubstituted naphthyl or 1,4-benzodioxan-5-yl, and p is 1.

PS33364/AT
SE07
RPS/KEB: 6th Jan 86

REGINALD PETER SLATCHER

AUTHORISED REPRESENTATIVE
General Authorisation No. 97

European Patent Office

# EUROPEAN SEARCH REPORT

0194750

Application number

EP 86 30 0883

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,A | JOURNAL OF MEDICINAL CHEMISTRY, vol. 24, no. 5, May 1981, pages 628-631, American Chemical Society, US; J.J. BALDWIN et al.: "Approaches to vasodilating/beta-adrenergic blocking agents: Examples of the dihydrolutidine type" * Page 629, compounds 4a-c; page 631, experimental section; pages 630-631, discussion * --- | 1-4,8-10 | C 07 D 211/90<br>C 07 D 405/12<br>C 07 D 401/12<br>C 07 D 417/12<br>C 07 D 413/04<br>A 61 K 31/44 |
| A | GB-A-1 409 865 (SCIENCE UNION) * Example V; claims * --- | 1-4,8-10 | |
| P,X D | CHEMICAL ABSTRACTS, vol. 104, no. 13, 31st March 1986, page 711, no. 109478d, Columbus, Ohio, US; & JP - A - 60 136 558 (TEIKOKU HORMONE Mfg. CO., LTD.) 20-07-1985 --- | 1-4,8-10 | **TECHNICAL FIELDS SEARCHED (Int. Cl 4)** |
| P,X D | EP-A-0 151 006 (YAMANOUCHI PHARM. CO.) * Whole document, especially examples 1-58 * ----- | 1-4,8-10 | C 07 D 211<br>C 07 D 405<br>C 07 D 401<br>C 07 D 417<br>C 07 D 413<br>A 61 K 31 |

The present search report has been drawn up for all claims

| Place of search | Date of completion of the search | Examiner |
|---|---|---|
| THE HAGUE | 26-05-1986 | NUYTS A.M.K.A. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82